(19) Europäisches Patentamt
European Patent Office
Office européen des brevets

(11) **EP 3 177 381 B1**

(12) **FASCICULE DE BREVET EUROPEEN**

(45) Date de publication et mention de la délivrance du brevet:
**09.01.2019 Bulletin 2019/02**

(21) Numéro de dépôt: **15752972.8**

(22) Date de dépôt: **04.08.2015**

(51) Int Cl.:
*B01D 15/18* [(2006.01)]  *B01D 53/02* [(2006.01)]
*B01J 20/12* [(2006.01)]  *B01J 20/18* [(2006.01)]
*B01J 20/30* [(2006.01)]  *B01J 20/28* [(2006.01)]
*C07C 7/13* [(2006.01)]

(86) Numéro de dépôt international:
**PCT/EP2015/067968**

(87) Numéro de publication internationale:
**WO 2016/020388 (11.02.2016 Gazette 2016/06)**

(54) **ADSORBANTS ZÉOLITHIQUES À FAIBLE TAUX DE LIANT ET À HAUTE SURFACE EXTERNE, LEUR PROCÉDÉ DE PRÉPARATION ET LEURS UTILISATIONS**

ZEOLITHADSORPTIONSMITTEL MIT GERINGEM BINDEMITTELGEHALT UND GROSSER EXTERNER OBERFLÄCHE, VERFAHREN ZUR HERSTELLUNG DAVON UND VERWENDUNGEN DAVON

ZEOLITE ADSORBENTS WITH LOW BINDER CONTENT AND LARGE EXTERNAL SURFACE AREA, METHOD FOR PREPARATION OF SAME AND USES THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **05.08.2014 FR 1457625**

(43) Date de publication de la demande:
**14.06.2017 Bulletin 2017/24**

(73) Titulaires:
• **ARKEMA FRANCE**
  **92700 Colombes (FR)**
• **IFP Energies nouvelles**
  **92500 Rueil-Malmaison (FR)**

(72) Inventeurs:
• **BOUVIER, Ludivine**
  **F-64300 Orthez (FR)**
• **LUTZ, Cécile**
  **F-64290 Gan (FR)**
• **LAROCHE, Catherine**
  **F-69390 Vernaison (FR)**
• **BAUDOT, Arnaud**
  **F-69390 Vernaison (FR)**

(74) Mandataire: **Casalonga**
  **Casalonga & Partners**
  **Bayerstraße 71/73**
  **80335 München (DE)**

(56) Documents cités:
**WO-A1-2015/032923**   **FR-A1- 2 925 366**
**FR-A1- 3 010 328**   **FR-A1- 3 010 402**
**US-A1- 2011 011 804**

EP 3 177 381 B1

**Description**

L'invention concerne des adsorbants zéolithiques sous forme d'agglomérés à faible taux de liant comprenant de la zéolithe faujasite à porosité hiérarchisée pour leurs utilisations dans des applications où le transfert de matière est un paramètre important, lesdits adsorbants présentant une haute surface externe typiquement supérieure à 20 $m^2.g^{-1}$, ladite surface externe étant associée à une population de mésopores déterminée par mesure d'adsorption d'azote. Le terme « associée » dans la phrase précédente indique que la population de mésopores contribue à la valeur mesurée de la surface externe, en plus de la surface externe des cristaux de zéolithe(s).

La présente invention concerne également un procédé de préparation desdits adsorbants zéolithiques, ainsi que leurs utilisations, notamment pour la séparation de mélanges gazeux ou liquides d'isomères, plus particulièrement des xylènes et notamment pour la production de *para*-xylène très pur à partir d'une charge d'hydrocarbures aromatiques contenant des isomères à 8 atomes de carbone.

L'utilisation d'adsorbants zéolithiques comprenant au moins de la zéolithe Faujasite (FAU) de type X ou Y et comprenant, outre des cations sodium, des ions baryum, potassium ou strontium, seuls ou en mélanges, pour adsorber sélectivement le *para*-xylène dans un mélange d'hydrocarbures aromatiques, est bien connue de l'art antérieur.

Les brevets US 3 558 730, US 3 558 732, US 3 626 020 et US 3 663 638 montrent que des adsorbants zéolithiques comprenant des aluminosilicates à base de sodium et de baryum (US 3 960 774) ou à base de sodium, de baryum et de potassium, sont efficaces pour la séparation du *para*-xylène présent dans des coupes aromatiques en C8 (coupes comprenant des hydrocarbures aromatiques à 8 atomes de carbone).

Les adsorbants décrits dans le brevet US 3 878 127 sont utilisés comme agents d'adsorption dans les procédés en phase liquide, de préférence de type contre-courant simulé, similaires à ceux décrits dans le brevet US 2 985 589 et qui s'appliquent, entre autres, aux coupes aromatiques en C8.

Dans les brevets listés ci-dessus, les adsorbants zéolithiques se présentent sous forme de cristaux à l'état de poudre ou sous forme d'agglomérés constitués majoritairement de poudre de zéolithe et jusqu'à 20% en poids de liant inerte.

La synthèse des zéolithes FAU s'effectue habituellement par nucléation et cristallisation de gels de silico-aluminates. Cette synthèse conduit à des cristaux (généralement sous forme de poudre) dont l'emploi à l'échelle industrielle est particulièrement malaisé (pertes de charges importantes lors des manipulations). On préfère alors les formes agglomérées de ces cristaux, sous forme de grains, de filés et autres agglomérés, ces dites formes pouvant être obtenues par extrusion, pastillage, atomisation et autres techniques d'agglomération connues de l'homme du métier. Ces agglomérés ne présentent pas les inconvénients inhérents aux matières pulvérulentes.

D'autre part, les cristaux de zéolithes sont le plus souvent préparés à partir de solutions aqueuses sodiques (par exemple solution aqueuse d'hydroxyde de sodium), et, si on le souhaite, les cations sodium peuvent être remplacés (échangés) en totalité ou en partie par d'autres cations, par exemple baryum ou baryum et potassium. Ces échanges cationiques peuvent être effectués avant et/ou après agglomération de la zéolithe pulvérulente avec le liant d'agglomération, selon des techniques classiques connues de l'homme du métier.

Les agglomérés, qu'ils soient sous forme de plaquettes, de billes, d'extrudés, et autres, sont en général constitués de cristaux de zéolithe(s), qui constituent l'élément actif (au sens de l'adsorption) et d'un liant d'agglomération. Ce liant d'agglomération est destiné à assurer la cohésion des cristaux entre eux dans la structure agglomérée, mais aussi doit permettre d'assurer une résistance mécanique suffisante auxdits agglomérés afin d'éviter, ou tout au moins de minimiser le plus possible, les risques de fractures, brisures ou cassures qui pourraient survenir lors de leurs utilisations industrielles pendant lesquelles les agglomérés sont soumis à de nombreuses contraintes, telles que vibrations, variations fortes et/ou fréquentes de pressions, mouvements et autres.

La préparation de ces agglomérés s'opère par exemple par empâtage de cristaux de zéolithe à l'état de poudre avec une pâte argileuse, dans des proportions de l'ordre de 80 à 90 % en poids de poudre de zéolithe pour 20% à 10% en poids de liant, puis mise en forme en billes, plaquettes ou extrudés, et traitement thermique à haute température pour cuisson de l'argile et réactivation de la zéolithe, le ou les échange(s) cationique(s), comme par exemple l'échange au baryum et éventuellement au potassium pouvant être effectué avant et/ou après l'agglomération de la zéolithe pulvérulente avec le liant.

On obtient des corps zéolithiques dont la granulométrie est de quelques millimètres, voire de l'ordre du millimètre, et qui, si le choix du liant d'agglomération et la granulation sont faits dans les règles de l'art, présentent un ensemble de propriétés satisfaisantes, en particulier de porosité, de résistance mécanique, de résistance à l'abrasion. Cependant, les propriétés d'adsorption de ces agglomérés sont évidemment réduites par rapport à la poudre active de départ en raison de la présence de liant d'agglomération inerte vis-à-vis de l'adsorption.

Divers moyens ont déjà été proposés pour pallier cet inconvénient du liant d'agglomération d'être inerte quant aux performances d'adsorption, parmi lesquels, la transformation de la totalité ou d'au moins une partie du liant d'agglomération en zéolithe active du point de vue de l'adsorption. Cette opération est maintenant bien connue de l'homme du métier, par exemple sous la dénomination de « zéolithisation ». Pour effectuer facilement cette opération, on utilise des liants zéolithisables, le plus souvent appartenant à la famille de la kaolinite, et de préférence préalablement calcinés à des températures généralement comprises entre 500°C et 700°C.

La demande de brevet FR 2 789 914 décrit un procédé de fabrication d'agglomérés de zéolithe X, de rapport atomique Si/Al compris entre 1,15 et 1,5, échangés au baryum et éventuellement au potassium, en agglomérant des cristaux de zéolithe X avec un liant, une source de silice et de la carboxyméthylcellulose, puis en zéolithisant le liant par immersion de l'aggloméré dans une liqueur alcaline. Après échange des cations de la zéolithe par des ions baryum (et éventuellement potassium) et activation, les agglomérés ainsi obtenus présentent, du point de vue de l'adsorption du para-xylène contenu dans les coupes aromatiques en C8, des propriétés améliorées par rapport à des adsorbants préparés à partir de la même quantité de zéolithe X et de liant, mais dont le liant n'est pas zéolithisé.

Le brevet US 7 812 208 (UOP) décrit un procédé de séparation du *para*xylène contenu dans des coupes aromatiques, utilisant un adsorbant de type « binderless », c'est-à-dire sans matériau amorphe ou avec une quantité inférieure à 2% en poids de matériau amorphe, à base de zéolithe X, ayant une taille moyenne de cristaux inférieure à 1,8 $\mu$m. Ces adsorbants sont obtenus après une étape de zéolithisation du liant.

La demande de brevet FR 2 925 366 décrit un adsorbant zéolithique aggloméré comprenant une zéolithe LSX (ce qui est une zéolithe FAU) échangée par des ions Ba ou Ba et K, comprenant un liant inerte en proportion inférieure ou égale à 20% en poids, de préférence 15% en poids, de la masse totale de l'aggloméré. La demande de brevet décrit également un procédé séparatif de récupération de para-xylène à partir de coupes d'isomères C$_8$ aromatiques en phase liquide ou gazeuse en employant ledit adsorbant zéolithique aggloméré.

Ces adsorbants présentent des propriétés de transfert et d'adsorption améliorées et ne contiennent pas, ou seulement en quantité inferieure à 2% en poids, et souvent inférieure à 0,5% en poids, de matériau amorphe ou non zéolithique. En revanche, aucune information n'est donnée sur la résistance mécanique de telles particules « binderless ». Ce document enseigne qu'une conversion totale du liant en zéolithe permettrait de maximiser la capacité d'adsorption. Toutefois les propriétés mécaniques ne semblent pas toujours conservées ou optimisées dans ce cas.

Ceci est par exemple confirmé par la demande de brevet FR 2 999 098 qui décrit un adsorbant zéolithique aggloméré à base de zéolithe X à petits cristaux de taille typiquement inférieure à 1,7 $\mu$m et qui présente des propriétés maximales de sélectivité vis-à-vis du *para-xylène* et de transfert de matière. Pour ce type d'adsorbant un compromis s'impose entre résistance mécanique maximale et capacité d'adsorption optimisée. Il ressort également à la lumière des exemples que même après une zéolithisation optimale, plus la taille des cristaux de zéolithe de départ est faible (exemple à 0,8 $\mu$m) plus la résistance mécanique des adsorbants agglomérés est faible.

Les procédés de préparation décrits dans l'art antérieur font intervenir une étape supplémentaire de zéolithisation qui, outre le fait de potentiellement dégrader la cristallinité des cristaux de taille réduite (< 0,5 $\mu$m), engendre des coûts supplémentaires.

Outre une capacité d'adsorption élevée et de bonnes propriétés de sélectivité vis-à-vis de l'espèce à séparer du mélange réactionnel, l'adsorbant doit présenter de bonnes propriétés de transfert de matière afin de garantir un nombre de plateaux théoriques suffisants pour réaliser une séparation efficace des espèces en mélange, comme l'indique Ruthven dans l'ouvrage intitulé « Principles of Adsorption and Adsorption Processes » (« Principes de l'Adsorption et des Procédés d'Adsorption »), John Wiley & Sons, (1984), pages 326 et 407. Ruthven indique (*ibid.,* page 243), que, dans le cas d'un adsorbant aggloméré, le transfert de matière global dépend de l'addition de la résistance diffusionnelle intra-cristalline et de la résistance diffusionnelle entre les cristaux.

La résistance diffusionnelle intra-cristalline est proportionnelle au carré des diamètres des cristaux et inversement proportionnelle à la diffusivité intracristalline des molécules à séparer.

La résistance diffusionnelle entre les cristaux (également appelée « résistance macroporeuse ») est quant à elle proportionnelle au carré des diamètres des agglomérés, inversement proportionnelle à la porosité contenue dans les macropores et mésopores (c'est-à-dire les pores dont l'ouverture est supérieure à 2 nm) au sein de l'aggloméré, et inversement proportionnelle à la diffusivité des molécules à séparer dans cette porosité.

La taille des agglomérés est un paramètre important lors de l'utilisation de l'adsorbant dans l'application industrielle, car elle détermine la perte de charge au sein de l'unité industrielle et l'uniformité du remplissage. La distribution granulométrique des agglomérés doit donc être étroite, et centrée sur des diamètres moyens en nombre compris typiquement entre 0,40 mm et 0,65 mm afin d'éviter des pertes de charges excessives.

La porosité contenue dans les macropores et mésopores peut être augmentée en utilisant des agents porogènes, tels que par exemple l'amidon de maïs conseillé dans le document US8283274 pour améliorer le transfert de matière. Cependant, cette porosité ne participe pas à la capacité d'adsorption et par conséquent l'amélioration du transfert de matière macroporeux se fait alors au détriment de la capacité d'adsorption volumique. Par conséquent, cette voie d'amélioration du transfert de matière macroporeux s'avère très limitée.

Pour estimer l'amélioration de la cinétique de transfert, il est possible d'utiliser la théorie des plateaux décrite par Ruthven dans « Principles of Adsorption and Adsorption Processes », ibid., pages 248-250. Cette approche est basée sur la représentation d'une colonne par un nombre fini de réacteurs hypothétiques idéalement agités (étages théoriques). La hauteur équivalente de plateaux théoriques est une mesure directe de la dispersion axiale et de la résistance au transfert de matière du système.

Pour une structure zéolithique donnée, une taille d'adsorbant donnée et une température de fonctionnement donnée, les diffusivités sont fixées, et un des moyens pour améliorer le transfert de matière consiste à réduire le diamètre des cristaux. Un gain sur le transfert de matière global sera ainsi obtenu en réduisant la taille des cristaux.

L'homme du métier va donc chercher à réduire le plus possible le diamètre des cristaux de zéolithes afin d'améliorer le transfert de matière.

Le brevet CN1267185C, revendique ainsi des adsorbants contenant 90% à 95% de zéolithe BaX ou BaKX pour la séparation du para-xylène, dans lesquels les cristaux de zéolithe X sont de taille comprise entre 0,1 $\mu$m et 0,4 $\mu$m et ceci afin d'améliorer les performances en transfert de matière. De même, la demande US2009/0326308 décrit un procédé de séparation des isomères du xylène dont les performances ont été améliorées par l'utilisation d'adsorbants à base de cristaux de zéolithe X de taille inférieure à 0,5 $\mu$m.

La demanderesse a néanmoins observé que la synthèse, la filtration, la manipulation et l'agglomération de cristaux de zéolithe dont la taille est inférieure à 0,5 $\mu$m mettent en oeuvre des procédés lourds, peu économiques et donc difficilement industrialisables.

De plus, de tels adsorbants comportant des cristaux de taille inférieure à 0,5 $\mu$m, s'avèrent également plus fragiles, et il devient alors nécessaire d'augmenter le taux de liant d'agglomération afin de renforcer la cohésion des cristaux entre eux au sein de l'adsorbant. Toutefois, l'augmentation du taux de liant d'agglomération conduit à une densification des adsorbants, à l'origine d'une augmentation de la résistance diffusionnelle macroporeuse. Ainsi, malgré une résistance diffusionnelle intra-cristalline réduite du fait de la diminution de la taille des cristaux, l'augmentation de la résistance diffusionnelle macroporeuse en raison de la densification de l'adsorbant, ne permet pas une amélioration du transfert global.

Par ailleurs, l'augmentation du taux de liant ne permet pas d'obtenir une bonne capacité d'adsorption.

La capacité finale d'adsorption peut être améliorée en effectuant comme enseigné dans l'art antérieur une zéolithisation du liant d'agglomération de l'adsorbant.

Toutefois l'effet bénéfique de cette étape de conversion du liant peut être fortement pénalisé par la dégradation de la cristallinité des nano-cristaux de départ, dégradation provoquée par les solutions basiques mises en jeu lors de cette étape de zéolithisation.

Une troisième propriété de l'adsorbant nécessaire pour garantir de bonnes performances du procédé de séparation en phase liquide de type contre-courant simulé est d'avoir une bonne résistance mécanique. En effet, en conditions standards de fonctionnement de ce type de procédé, une contrainte mécanique importante s'applique sur l'adsorbant au sein des unités industrielles, entraînant la formation de particules fines, qui induisent une détérioration des performances (voir par exemple « Primary Analysis on State of Xylene Adsorption Unit », Li et coll., Jingxi Shiyou Huagong, 2004, (4), 54-55), et ce d'autant plus que la résistance mécanique de l'adsorbant sera faible.

Cependant, l'art antérieur FR2999098 montre que lorsque des cristaux de petites tailles (exemple à 0,8 $\mu$m) sont utilisés, la résistance mécanique diminue également et ce malgré l'étape de zéolithisation. L'homme de l'art tendrait donc à augmenter la taille des cristaux afin d'améliorer la résistance mécanique.

En résumé, pour la séparation des xylènes l'art antérieur montre qu'il faut :

- 1) diminuer la taille des cristaux pour améliorer le transfert de matière,
- 2) et/ou augmenter la macroporosité par l'utilisation d'agents porogènes et
- 3) zéolithiser le liant pour augmenter la résistance mécanique et maximiser la capacité d'adsorption.

Il semble donc difficile d'obtenir des adsorbants avec toutes les propriétés suivantes réunies :

- un transfert de matière au sein de l'adsorbant le plus rapide possible, c'est-à-dire une résistance au transfert de matière la plus faible possible et idéalement quasi nulle, voire nulle,
- une résistance mécanique à l'écrasement optimale,
- une capacité d'adsorption la plus grande possible (i.e. une teneur en zéolithe (phase cristalline active au sens de l'adsorption) la plus grande possible).

Les inventeurs ont maintenant découvert que les problèmes rencontrés dans l'art antérieur peuvent être solutionnés en totalité ou au moins en partie grâce aux adsorbants selon la présente invention.

En particulier, un but de la présente invention consiste à fournir des adsorbants dont le transfert de matière a été maximisé grâce à l'utilisation de zéolithes :

- à porosité hiérarchisée,
- de taille de cristaux dite conventionnelle, c'est-à-dire de diamètre moyen en nombre supérieur ou égal à 1 $\mu$m,
- et dont le taux de liant (phase non zéolithique) a été optimisé afin de maximiser la capacité d'adsorption tout en conservant des propriétés mécaniques compatibles avec leur utilisation dans les procédés d'adsorption.

Dans ce qui va suivre, et à moins d'une autre indication, les bornes d'un domaine de valeurs sont comprises dans ce domaine, notamment dans les expressions « compris entre » et « allant de ... à ... ».

Par « zéolithe à porosité hiérarchisée », on entend une zéolithe possédant à la fois des micropores et des mésopores, autrement dit une zéolithe à la fois microporeuse et mésoporeuse. Par « zéolithe mésoporeuse », on entend une zéolithe dont les cristaux zéolithiques microporeux présentent, conjointement à la microporosité, des cavités internes de taille nanométrique (mésoporosité), facilement identifiables par observation au moyen d'un Microscope Électronique à Transmission (MET ou « TEM » en langue anglaise), comme décrit par exemple dans US 7 785 563.

Les adsorbants zéolithiques selon l'invention présentent une importante surface externe associée à une population de mésopores de diamètre moyen compris entre 2 nm et 50 nm, caractérisés par adsorption d'azote.

Le diamètre moyen des mésopores est déterminé par la méthode Barrett-Joyner-Halenda (méthode BJH, E. P.Barrett, L. G. Joyner, P. P. Halenda, "The Determination of Pore Volume and Area Distributions in Porous Substances. I. Computations from Nitrogen Isotherms", J. Am. Chem. Soc., 73(1), (1951), 373-380), à partir de la branche d'adsorption de l'isotherme de physisorption d'azote à 77K.

Avantageusement, la distribution en volume de diamètre moyen ainsi déterminé des mésopores de l'adsorbant selon l'invention, représentée graphiquement par $dV/dDm$ ou $dV/d\log Dm$ en fonction du diamètre moyen Dm, correspond à une distribution unimodale et étroite.

Par « distribution unimodale », on entend une distribution ne présentant qu'un seul pic. Une distribution unimodale de diamètre moyen est ainsi caractérisée par un seul pic, pour lequel la valeur du diamètre moyen au sommet du pic, est appelée « mode » ou encore « valeur dominante », et représente la valeur la plus fréquente de la distribution. Lorsqu'une distribution présente deux pics séparés par un creux, on dit que la distribution est bimodale. L'invention ne concerne pas le cas de distribution bimodale voire multimodale, c'est-à-dire de distribution où il existe plusieurs zones de concentration des valeurs séparées par des discontinuités. De telles distributions sont caractéristiques de la présence de plusieurs populations de pores de diamètres moyens distincts.

Le terme « étroite », utilisé pour caractériser la distribution de diamètre moyen des mésopores, indique que la largeur à mi-hauteur de la distribution autour du mode est inférieure à 20 nm, de préférence inférieure à 15 nm, de manière préférée comprise entre 10 nm et 0,1 nm et de manière encore préférée comprise entre 5 nm et 0,5 nm, comme décrit plus loin dans les techniques de caractérisation.

L'utilisation de cristaux de zéolithe à porosité hiérarchisée de taille dite « conventionnelle » permet à la fois :

- d'utiliser des cristaux de taille micrométrique (c'est-à-dire de diamètre moyen en nombre supérieur ou égal à 1 $\mu$m) mais avec des propriétés de transfert équivalentes à celles obtenues avec de très petits cristaux nanométriques, typiquement de diamètre moyen en nombre strictement inférieur à 0,5 $\mu$m, et
- de conserver, pour l'adsorbant obtenu à partir de ces zéolithes à porosité hiérarchisée, une résistance mécanique adaptée à son utilisation dans des procédés de séparation, avec un taux de liant(s) réduit qui permet de maintenir un niveau élevé en terme de capacité d'adsorption sans avoir nécessairement recours à la zéolithisation du liant.

Comme indiqué précédemment, ces zéolithes à porosité hiérarchisée sont à la fois microporeuses et mésoporeuses, ces termes ayant été définis plus haut dans la description. Comme indiqué dans US7785563, l'observation par microscopie électronique à transmission (MET) permet de vérifier si les cristaux zéolithiques de l'adsorbant sont des cristaux de zéolithe pleins (i.e. non mésoporeux) ou des agrégats de cristaux de zéolithes pleins ou des cristaux mésoporeux.

Un autre but de la présente invention consiste à fournir un procédé de préparation desdits adsorbants, et en particulier un procédé de préparation desdits adsorbants plus économique que les procédés décrits dans l'art antérieur, ainsi que les utilisations desdits adsorbants pour la séparation de mélanges gazeux ou liquides d'isomères, plus particulièrement des xylènes et notamment pour la séparation de *para*-xylène très pur à partir d'une charge d'hydrocarbures aromatiques contenant des isomères à 8 atomes de carbone.

Un autre but encore de la présente invention consiste à maximiser le transfert de matière au sein de l'adsorbant zéolithique, tout en maintenant une capacité d'adsorption convenable pour l'application, en même temps qu'une résistance mécanique compatible avec l'application considérée.

Comme autre but encore, la présente invention propose un adsorbant zéolithique optimisé pour utilisation dans des procédés de séparation alliant une bonne résistance mécanique, une capacité d'adsorption élevée et un transport des molécules au sein de l'adsorbant et de la phase zéolithique maximisé (transfert de matière maximisé).

Ainsi, et selon un premier aspect, la présente invention concerne un adsorbant zéolithique comprenant au moins une zéolithe FAU à porosité hiérarchisée et comprenant du baryum ou du baryum et du potassium, adsorbant zéolithique pour lequel :

- la surface externe, mesurée par adsorption d'azote, est supérieure à 20 $m^2.g^{-1}$, de préférence supérieure à 30 $m^2.g^{-1}$, et de préférence encore comprise entre 30 $m^2.g^{-1}$ et 200 $m^2.g^{-1}$, et plus préférentiellement comprise entre 30 $m^2.g^{-1}$ et 150 $m^2.g^{-1}$, ladite surface externe étant associée à une population de mésopores de diamètre moyen compris entre 2 nm et 50 nm, et

- le taux de phase non zéolithique (phase inerte au sens de l'adsorption) est compris entre 6% et 12% en poids par rapport au poids total de l'adsorbant, de préférence compris entre 6% et 11% en poids par rapport au poids total de l'adsorbant, de préférence encore entre 6% et 10%, en poids par rapport au poids total de l'adsorbant.

Dans un mode de réalisation préféré de l'invention, la zéolithe FAU à porosité hiérarchisée de l'adsorbant zéolithique est une zéolithe pour laquelle :

- le diamètre moyen en nombre des cristaux est compris entre 1 $\mu$m et 20 $\mu$m, de préférence encore compris entre 1,5 $\mu$m et 20 $\mu$m, plus préférentiellement entre 1,8 $\mu$m et 10 $\mu$m, mieux encore entre 2 $\mu$m et 10 $\mu$m, et de préférence encore entre 2 $\mu$m et 8 $\mu$m,

- la surface externe des cristaux, mesurée par adsorption d'azote, supérieure à 40 $m^2.g^{-1}$, de préférence comprise entre 40 $m^2.g^{-1}$ et 200 $m^2.g^{-1}$, de préférence encore comprise entre 40 $m^2.g^{-1}$ et 150 $m^2.g^{-1}$.

La surface externe de l'adsorbant zéolithique de l'invention est calculée par la méthode du t-plot à partir de l'isotherme d'adsorption d'azote à une température de 77K, après dégazage sous vide (P < 6,7.10$^{-4}$ Pa), à une température comprise entre 300°C et 450°C pendant une durée allant de 9 heures à 16 heures, de préférence à 400°C pendant 10 heures. La surface externe de la zéolithe FAU à porosité hiérarchisée est mesurée de la même manière.

Selon un aspect préféré, la teneur en baryum (Ba) de l'adsorbant zéolithique de l'invention, exprimée en oxyde de baryum (BaO), est supérieure à 10%, de préférence supérieure à 15%, de manière très préférée supérieure à 20%, de manière encore plus préférée supérieure à 23 %, voire supérieure à 33% en poids par rapport à la masse totale de l'adsorbant, et avantageusement, la teneur en baryum est comprise entre 23% et 42%, et typiquement entre 30% et 40%, en poids par rapport au poids total de l'adsorbant.

Selon un autre aspect préféré, la teneur en potassium (K) de l'adsorbant zéolithique de l'invention, exprimée en oxyde de potassium ($K_2O$), est inférieure à 25%, de préférence comprise entre 0% et 20%, de manière encore plus préférée comprise entre 0% et 15% et de manière très préférée de 0% à 10% en poids par rapport à la masse totale de l'adsorbant.

Selon encore un autre mode de réalisation préféré, la teneur totale en ions alcalins ou alcalino-terreux, autres que baryum et potassium, exprimée en teneur totale d'oxydes d'ions alcalins ou alcalino-terreux autres que l'oxyde de baryum BaO et l'oxyde de potassium $K_2O$, est comprise entre 0 et 5%, par rapport à la masse totale de l'adsorbant.

De manière préférée, la population de mésopores dudit adsorbant zéolithique présente des diamètres moyens au mode, caractérisés par adsorption d'azote compris entre 2 nm et 30 nm et de préférence compris entre 2 nm et 20 nm.

De manière avantageuse, l'adsorbant zéolithique selon l'invention présente un volume total contenu dans les macropores et les mésopores (somme du volume macroporeux et du volume mésoporeux) mesuré par intrusion de mercure, compris entre 0,15cm$^3.g^{-1}$ et 0,5cm$^3.g^{-1}$, de préférence compris entre 0,20 cm$^3.g^{-1}$ et 0,40 cm$^3.g^{-1}$ et de manière très préférée compris entre 0,20 cm$^3.g^{-1}$ et 0,35 cm$^3.g^{-1}$.

Selon un mode de réalisation préféré de la présente invention, l'adsorbant zéolithique comprend à la fois des macropores, des mésopores et des micropores. Par « macropores », on entend des pores dont le diamètre est supérieur à 50 nm, de préférence compris entre 50 nm et 400 nm. Par « mésopores », on entend des pores dont le diamètre est compris entre 2 nm et 50 nm. Par « micropores », on entend des pores dont le diamètre est inférieur à 2 nm.

En outre, l'adsorbant de l'invention présente avantageusement un ratio (volume macroporeux)/(volume macroporeux + volume mésoporeux) compris entre 0,2 et 1, de manière très préférée compris entre 0,4 et 0,8.

On préfère également, dans le cadre de la présente invention, un adsorbant zéolithique dont le volume microporeux, évalué par la méthode de t-plot à partir de l'isotherme d'adsorption d'azote ($N_2$) à une température de 77K, est supérieur à 0,200 cm$^3.g^{-1}$, de préférence compris entre 0,205 cm$^3.g^{-1}$ et 0,270 cm$^3.g^{-1}$ et de préférence encore compris entre 0,205 cm$^3.g^{-1}$ et 0,260 cm$^3.g^{-1}$. Ladite mesure de volume microporeux est calculée après dégazage sous vide (P < 6,7.10$^{-4}$ Pa), à une température comprise entre 300°C et 450°C pendant une durée allant de 9 heures à 16 heures, de préférence à 400°C pendant 10 heures.

Dans le cadre de la présente invention, la résistance mécanique est mesurée par la méthode Shell série SMS1471-74 adaptée pour des agglomérés de taille inférieure à 1,6 mm. Cette résistance mécanique, mesurée pour l'adsorbant zéolithique défini précédemment, est généralement comprise entre 1,5 MPa et 4 MPa, de préférence entre 1,7 MPa et 4 MPa de préférence encore entre 1,8 MPa et 4 MPa et de manière tout à fait préférée entre 2 MPa et 4 MPa.

Selon encore un autre mode de réalisation préféré, l'adsorbant zéolithique selon l'invention présente un rapport atomique Si/Al compris entre 1,00 et 2,00 de préférence entre 1,00 et 1,80, de préférence encore entre 1,15 et 1,80, et de manière encore plus préférée entre 1,15 et 1,60.

Parmi les zéolithes FAU de type X, il est maintenant communément admis de reconnaître entre autres deux sous-groupes dénommés zéolithes LSX et zéolithes MSX. Les zéolithes LSX présentent un rapport atomique Si/Al égal à environ 1 et les zéolithes MSX présentent un rapport atomique Si/Al compris entre environ 1,05 et environ 1,15. Selon un mode de réalisation préféré, la au moins une zéolithe FAU est une zéolithe X de rapport atomique Si/Al compris entre 1,10 et 1,50. Selon un autre mode de réalisation préféré, la au moins une zéolithe X est une zéolithe de type LSX de rapport atomique Si/Al est égal à environ 1.

Dans l'adsorbant zéolithique de la présente invention, et selon un mode de réalisation préféré, par « zéolithe FAU à porosité hiérarchisée », on entend les zéolithes FAU de type X définies ci-dessus, ces dites zéolithes étant à porosité hiérarchisée c'est-à-dire, les zéolithes de type X à porosité hiérarchisée (ou zéolithe XPH), les zéolithes de type MSX à porosité hiérarchisée (ou MSXPH) et les zéolithes de type LSX à porosité hiérarchisée (ou LSXPH), et plus particulièrement les zéolithes FAU à porosité hiérarchisée et de rapport atomique Si/Al compris entre 1,00 et 1,50, de préférence entre 1,05 et 1,50, de préférence encore entre 1,05 et 1,40, et de manière encore plus préférée, entre 1,10 et 1,40.

L'invention comprend également les adsorbants zéolithiques comprenant des mélanges de deux ou plusieurs zéolithes FAU à porosité hiérarchisée telles qu'elles viennent d'être définies.

Selon un autre mode de réalisation préféré, aucune structure zéolithique autre que la structure FAU, de préférence aucune structure zéolithique autre que la structure faujasite X, n'est détectée par diffraction des rayons X (connue de l'homme du métier sous l'acronyme DRX) dans l'adsorbant zéolithique de la présente invention.

Par aucune structure zéolithique autre que la structure FAU on entend moins de 2% en poids, borne incluse, d'une ou de plusieurs autres phases zéolithiques autres que la structure FAU (fraction massique déterminée par DRX, technique décrite ci-après).

L'adsorbant zéolithique selon l'invention comprend en outre et de préférence au moins une Phase Non Zéolithique (PNZ) qui comprend entre autres un liant d'agglomération utilisé dans le mode de préparation pour assurer la cohésion des cristaux entre eux, d'où le terme « aggloméré » ou « aggloméré zéolithique » employé parfois en lieu et place du terme « adsorbant zéolithique » de l'invention, tel que décrit précédemment.

Dans la présente invention, le terme « liant » signifie un liant d'agglomération qui permet d'assurer la cohésion des cristaux de zéolithe(s) dans l'adsorbant zéolithique (ou matériau zéolithique aggloméré) de l'invention. Ce liant se distingue en outre des cristaux de zéolithe(s) en ce qu'il ne présente pas de structure cristalline zéolithique après calcination, raison pour laquelle le liant est souvent qualifié d'inerte, et plus précisément inerte vis-à-vis de l'adsorption et de l'échange ionique.

Selon encore un autre mode de réalisation préféré, la fraction massique de zéolithe FAU, la zéolithe FAU étant de préférence une zéolithe de type X, est supérieure ou égale à 88% par rapport au poids total d'adsorbant de la présente invention, le complément à 100% étant de préférence constitué de phase non zéolithique (PNZ).

Comme déjà indiqué, la fraction massique de zéolithe(s) (taux de cristallinité) de l'adsorbant selon l'invention peut être déterminée par analyse par diffraction de rayons X, connue de l'homme du métier sous l'acronyme DRX.

Selon un mode de réalisation préféré, l'adsorbant zéolithique selon l'invention présente une perte au feu, mesurée à 950°C selon la norme NF EN 196-2, inférieure ou égale à 7,7%, de préférence comprise entre 0 et 7,7%, de manière préférée entre 3,0 et 7,7%, de manière encore préférée entre 3,5% et 6,5% et avantageusement entre 4,5% et 6%.

Un autre objet de l'invention concerne un procédé de préparation de l'adsorbant zéolithique tels qu'il vient d'être défini, ledit procédé comprenant au moins les étapes de :

a) agglomération de cristaux d'au moins une zéolithe de type FAU à porosité hiérarchisée, présentant une surface externe supérieure à 40 $m^2.g^{-1}$, de préférence comprise entre 40 $m^2.g^{-1}$ et 200 $m^2.g^{-1}$, de préférence encore comprise entre 40 $m^2.g^{-1}$ et 150 $m^2.g^{-1}$, dont le diamètre moyen en nombre des cristaux est compris entre 1 μm et 20 μm, de préférence encore compris entre 1,5 μm et 20 μm, plus préférentiellement 1,8 μm et 10μm, mieux encore entre 2 μm et 10 μm, et de préférence encore entre 2 μm et 8 μm, avec un liant comprenant de préférence au moins 80% d'argile ou d'un mélange d'argiles et avec jusqu'à 5% d'additifs ainsi qu'avec la quantité d'eau qui permet la mise en forme du matériau aggloméré, puis séchage et calcination des agglomérats ;

b) échange(s) cationique(s) des agglomérats de l'étape a) par mise en contact avec une solution d'ions baryum et/ou d'ions baryum et d'ions potassium ;

c) échange cationique éventuel supplémentaire des agglomérats de l'étape b) par mise en contact avec une solution d'ions potassium ;

d) lavage et séchage des agglomérats obtenus aux étapes b) ou c), à une température comprise entre 50°C et 150°C ; et

e) obtention de l'adsorbant zéolithique selon l'invention par activation des agglomérats obtenus à l'étape d) sous balayage gazeux oxydant et/ou inerte, avec notamment des gaz tels que l'oxygène, l'azote, l'air, un air sec et/ou décarbonaté, un air appauvri en oxygène, éventuellement sec et/ou décarbonaté, à une température comprise entre 100°C et 400°C, de préférence entre 200°C et 300°C pendant une durée déterminée en fonction de la teneur en eau et de la perte au feu souhaitées, typiquement de 1 à 6 heures.

Dans un mode de réalisation préféré du procédé de préparation de l'adsorbant zéolithique de la présente invention, le séchage des agglomérats à l'étape a) ci-dessus est généralement réalisé à une température comprise entre 50°C et 150°C, et la calcination des agglomérats séchés est généralement réalisée sous balayage gazeux oxydant et/ou inerte, avec notamment des gaz tels que l'oxygène, l'azote, l'air, un air sec et/ou décarbonaté, un air appauvri en oxygène, éventuellement sec et/ou décarbonaté, à une température supérieure à 150°C, typiquement comprise entre 180°C et 800°C, préférentiellement entre 200°C et 650°C, pendant quelques heures, par exemple de 2 heures à 6 heures.

Selon un mode de réalisation préféré, ladite au moins une zéolithe FAU est telle que définie et présente avantageusement un rapport atomique Si/Al compris de préférence entre 1,00 et 1,50, de préférence entre 1,05 et 1,50, de préférence encore entre 1,05 et 1,40, et de manière encore plus préférée compris entre 1,10 et 1,40.

Comme indiqué précédemment, la surface externe des cristaux mis en oeuvre dans l'étape a) du procédé décrit ci-dessus est calculée par la méthode du t-plot à partir de l'isotherme d'adsorption d'azote à une température de 77K, après dégazage sous vide (P < 6,7.10$^{-4}$ Pa), à une température comprise entre 300°C et 450°C pendant une durée allant de 9 heures à 16 heures, de préférence à 400°C pendant 10 heures.

Les cristaux de zéolithe FAU à porosité hiérarchisée présentant une importante surface externe peuvent être obtenus selon diverses méthodes connues de l'homme du métier et par exemple selon la synthèse décrite par Inayat et coll. (Angew. Chem. Int. Ed., (2012), 51, 1962-1965).

Il est également possible de préparer lesdits cristaux par synthèse par ensemencement et/ou par ajustement des conditions opératoires de synthèse tels que le rapport SiO$_2$/Al$_2$O$_3$, la teneur en sodium et l'alcalinité du mélange de synthèse ou encore selon des procédés de post-traitement de cristaux de zéolithe FAU conventionnels et connus de l'homme du métier.

Les procédés de post-traitements consistent généralement à éliminer des atomes du réseau zéolithique déjà formé, soit par un ou plusieurs traitements acides qui désaluminent le solide, traitement(s) suivi(s) par un ou plusieurs lavage(s) à la soude (NaOH) afin d'éliminer les résidus aluminiques formés, comme décrit par exemple par D. Verboekend et coll. (Adv. Funct. Mater., 22, (2012), pp. 916-928), soit encore par des traitements qui associent l'action d'un acide et celle d'un agent structurant qui améliorent l'efficacité du traitement acide, comme décrit par exemple dans la demande WO2013/106816.

Les procédés de synthèse directe de ces zéolithes (c'est-à-dire des procédés de synthèse autre que le post-traitement) sont préférés et font généralement intervenir un ou plusieurs agents structurants ou gabarits sacrificiels.

Les gabarits sacrificiels pouvant être utilisés peuvent être de tout type connu de l'homme du métier et notamment ceux décrits dans la demande WO2007/043731. Selon un mode de réalisation préféré, le gabarit sacrificiel est avantageusement choisi parmi les organosilanes et plus préférentiellement parmi le chlorure de [3-(triméthoxysilyl)propyl]octadécyldiméthylammonium, le chlorure de [3-(triméthoxysilyl)propyl]hexadécyldiméthylammonium, le chlorure de [3-(triméthoxysilyl)propyl]dodécyldiméthylammonium, le chlorure de [3-(triméthoxysilyl)propyl]octylammonium, la N-[3-(triméthoxysilyl)-propyl]aniline, le 3-[2-(2-amino-éthylamino)éthylamino]propyltriméthoxysilane, la N-[3-(triméthoxysilyl)propyl]-N'-(4-vinylbenzyl)éthylènediamine, le triéthoxy-3-(2-imidazolin-1-yl)propylsilane, la 1-[3-(triméthoxysilyl)propyl]urée, la N-[3-(triméthoxysilyl)propyl]-éthylènediamine, le [3-(diéthylamino)propyl]triméthoxysilane, le (3-glycidyloxypropyl)triméthoxysilane, le méthacrylate de 3-(triméthoxysilyl)propyle, le [2-(cyclohexényl)éthyl]triéthoxysilane, le dodécyltriéthoxysilane, l'hexadécyltriméthoxysilane, le (3-aminopropyl)triméthoxysilane, le (3-mercaptopropyl)triméthoxysilane, le (3-chloropropyl)triméthoxysilane, ainsi que les mélanges de deux ou plusieurs d'entre eux en toutes proportions.

Parmi les gabarits sacrificiels listés ci-dessus, on préfère tout particulièrement le chlorure de [3-(triméthoxysilyl)propyl]octadécyldiméthylammonium, ou TPOAC.

On peut également utiliser des gabarits sacrificiels de masse molaire plus élevée et par exemple les PPDA (Polymer Poly-DiallyldimethylAmmonium), PVB (PolyVinyl Butyral) et autres composés oligomères connus dans le domaine pour augmenter le diamètre des mésopores.

Selon un mode de réalisation préféré du procédé de la présente invention, on procède, à l'étape a), à l'agglomération de cristaux d'au moins une zéolithe FAU à porosité hiérarchisée, comme décrit précédemment, préparée en présence d'un gabarit sacrificiel destiné à être éliminé.

Cette élimination peut être réalisée selon les méthodes connues de l'homme du métier, par exemple par calcination, et de manière non limitative, la calcination des cristaux de zéolithe comprenant le gabarit sacrificiel peut être effectuée sous balayage gazeux oxydant et/ou inerte, avec notamment des gaz tels que l'oxygène, l'azote, l'air, un air sec et/ou décarbonaté, un air appauvri en oxygène, éventuellement sec et/ou décarbonaté, à une ou des températures supérieures à 150°C, typiquement comprises entre 180°C et 800°C, préférentiellement entre 200°C et 650°C, pendant quelques heures, par exemple entre 2 et 6 heures. La nature des gaz, les rampes de montée en température et les paliers successifs de températures, leurs durées seront adaptées en fonction de la nature du gabarit sacrificiel.

L'étape supplémentaire d'élimination de l'éventuel gabarit sacrificiel peut être effectuée à tout moment au cours du procédé de préparation de l'adsorbant zéolithique de l'invention. L'élimination dudit gabarit sacrificiel peut ainsi avantageusement être effectuée par calcination des cristaux de zéolithe avant l'étape d'agglomération a), ou encore de manière concomitante avec la calcination de l'adsorbant lors de l'étape a).

On ne sortirait toutefois pas du cadre de l'invention si l'agglomération de l'étape a) comprenait l'agglomération de plusieurs zéolithes FAU à porosité hiérarchisée obtenues selon des modes différents.

La synthèse de zéolithe de type FAU se fait généralement en milieu sodique (hydroxyde de sodium et donc cation $Na^+$). Les cristaux de zéolithe FAU ainsi obtenus comprennent majoritairement, voire exclusivement des cations sodium. On ne sortirait toutefois pas du cadre de l'invention en utilisant des cristaux ayant subi un ou plusieurs échanges cationiques, entre la synthèse sous forme Na, avant ou après l'élimination éventuelle du gabarit sacrificiel si cette étape est opérée avant la mise en oeuvre à l'étape a). Dans ce cas, l'étape b) et éventuellement l'étape c) d'échange devien(nen)t par conséquent éventuellement non nécessaire(s).

La taille des cristaux de zéolithe FAU utilisés à l'étape a) et des cristaux de zéolithe FAU dans les adsorbants selon l'invention est mesurée par observation au microscope électronique à balayage (MEB). Comme indiqué précédemment, de manière préférée, le diamètre moyen en nombre des cristaux est compris entre 1 $\mu$m et 20 $\mu$m, de préférence encore compris entre 1,5 $\mu$m et 20 $\mu$m, plus préférentiellement 1,8 $\mu$m et 10 $\mu$m, mieux encore entre 2 $\mu$m et 10 $\mu$m, et de préférence encore entre 2 $\mu$m et 8 $\mu$m. Cette observation MEB permet également de confirmer la présence de phase non zéolithique comprenant par exemple du liant d'agglomération ou toute autre phase amorphe dans les adsorbants.

Dans le présent document, on emploie l'appellation « diamètre moyen en nombre » ou bien « taille », notamment pour les cristaux de zéolithe. La méthode de mesure de ces grandeurs est explicitée plus loin dans la description.

L'agglomération et la mise en forme (étape a) peuvent être réalisées selon toutes les techniques connues de l'homme de l'art, et en particulier selon une ou plusieurs des techniques choisies parmi extrusion, compactage, agglomération sur assiette granulatrice, tambour granulateur, atomisation et autres.

Les proportions de liant d'agglomération (voir définition plus loin) et de zéolithe mises en oeuvre sont de 8 parties à 15 parties en poids de liant pour 92 parties à 85 parties en poids de zéolithe. Les adsorbants issus de l'étape a), qu'ils soient sous forme de billes, d'extrudés ou autres, ont de préférence un diamètre moyen en volume, ou leur longueur (plus grande dimension lorsqu'ils ne sont pas sphériques), comprise entre 0,2 mm et 2 mm, et en particulier comprise entre 0,2 mm et 0,8 mm et de préférence entre 0,40 mm et 0,65 mm.

À l'issue de l'étape a) les adsorbants agglomérés les plus fins peuvent être éliminés par cyclonage et/ou tamisage et/ou les agglomérés trop gros par tamisage ou concassage, dans le cas d'extrudés, par exemple.

Avantageusement, le liant d'agglomération n'est pas zéolithisé. Le liant utilisable dans le cadre de la présente invention peut donc être choisi parmi les liants classiques connus de l'homme du métier, zéolithisables ou non zéolithisables, et de préférence choisis parmi les argiles et les mélanges d'argiles, les silices, les alumines, les silices colloïdales, les gels d'alumine, et autres, et leurs mélanges.

Les argiles sont de préférence choisies parmi : kaolins, kaolinites, nacrites, dickites, halloysites, attapulgites, sépiolites, montmorillonites, bentonites, illites et métakaolins, ainsi que les mélanges de deux ou plusieurs d'entre elles en toutes proportions.

Préférence est donnée aux argiles fibreuses de type sépiolite ou attapulgite, l'argile ou les argiles pouvant, de manière générale, être formulée(s) sous forme de poudres broyées à sec et sélectées, ou mieux de gel (i.e. argiles délaminées) et dispersées, et éventuellement broyées, tels que les argiles commerciales Min-U-Gel®, Pansil®, Pangel®, Cimsil®, Attagel®, Actigel®, etc., ayant ou non subi un ou plusieurs traitements chimiques. De tels gels sont par exemples décrits dans EP170299 ou US6743745.

Lors de l'étape a), outre le ou les cristaux de zéolithe(s), le liant peut comprendre également un ou plusieurs additifs. Les additifs sont préférentiellement organiques, par exemple de la lignine, de l'amidon, de la carboxyméthylcellulose, des molécules tensio-actives (cationiques, anioniques, non ioniques ou amphotères), destinées à faciliter la manipulation de la pâte zéolithe(s)/argile(s) par modification de la rhéologie et/ou du pouvoir collant ou à conférer aux adsorbants finaux des propriétés satisfaisantes, notamment de macroporosité.

On peut citer de manière préférentielle mais non exhaustive, les méthyl-celluloses et leurs derivés, les lignosulfonates, les acides polycarboxyliques et les acides de copolymères carboxyliques, leurs derivés aminés et leurs sels, notamment les sels alcalins et les sels d'ammonium. Les additifs sont introduits à raison de 0 à 5%, de préférence de 0,1% à 2%, en poids par rapport au poids total de l'adsorbant.

Les additifs peuvent aussi être une source de silice liquide et/ou solide, de préférence représentant de 1% à 5% de la masse totale desdits solides. La source éventuelle de silice peut être de tout type connu de l'homme du métier, spécialiste de la synthèse de zéolithes, par exemple de la silice colloïdale, des diatomées, de la perlite, des cendres de calcination (« fly ash » en langue anglaise), du sable, ou toute autre forme de silice solide.

Pour la calcination comprise dans l'étape a), la nature des gaz, les rampes de montée en température et les paliers successifs de températures, ainsi que leurs durées respectives, seront adaptés notamment en fonction de la nature du gabarit sacrificiel à éliminer et en fonction de la nature du liant mis en oeuvre à l'étape d'agglomération a).

Les étapes d'échange(s) cationique(s) b) et c) décrites plus haut s'effectuent selon les méthodes classiques connues de l'homme du métier, et le plus souvent par mise en contact des adsorbants issus de l'étape a) avec un sel de baryum et/ou de baryum et de potassium, tel que le chlorure de baryum ($BaCl_2$) et/ou de potassium (KCl), en solution aqueuse à une température comprise entre la température ambiante et 100°C, et de préférence comprise entre 80°C et 100°C afin d'obtenir rapidement des teneurs élevées en baryum, i.e. des teneurs de préférence supérieures à 10%, de préférence supérieures à 15%, de manière très préférée supérieures à 20%, de manière encore plus préférée supérieures à 23%, voire supérieures à 33%, exprimées en poids d'oxyde de baryum par rapport à la masse totale de l'adsorbant.

Avantageusement, la teneur en baryum exprimée en oxyde de baryum est comprise entre 23% et 42%, et typiquement entre 30% et 40%, en poids par rapport au poids total de l'adsorbant. On préfère opérer avec un large excès d'ions baryum par rapport aux cations de la zéolithe que l'on souhaite échanger, typiquement un excès de l'ordre de 10 à 12, avantageusement en procédant par échanges successifs.

Un échange éventuel au potassium à l'étape c) peut être pratiqué avant et/ou après l'échange au baryum (étape b). Comme indiqué précédemment, il est également possible d'agglomérer à l'étape a) des cristaux de zéolithe FAU contenant déjà des ions baryum ou potassium ou baryum et potassium (pré-échange des cations présents dans la zéolithe de type FAU de départ, typiquement cations sodium, par des ions baryum ou potassium ou baryum et potassium avant l'étape a) et s'affranchir (ou non) des étapes b) et/ou c).

De manière surprenante, la demanderesse a observé que l'étape d'échange cationique, qui peut être délicate en raison de la relative fragilité de la structure des cristaux de zéolite à porosité hiérarchisée n'affecte pas les propriétés intrinsèques de surface externe et du volume microporeux (ramené à la masse de l'adsorbant une fois échangé) desdits cristaux de zéolithe à porosité hiérarchisée.

Après la ou les étape(s) d'échange(s) cationique(s), on procède ensuite à un lavage, généralement et de préférence à l'eau, puis d'un séchage de l'adsorbant ainsi obtenu.

L'activation qui suit le séchage, est conduite de manière classique, selon les méthodes connues de l'homme du métier, par exemple à une température en général comprise entre 100°C et 400°C, de préférence entre 200°C et 300°C pendant une durée déterminée en fonction de la teneur en eau et de la perte au feu souhaitées, typiquement de 1 à 6 heures.

La présente invention concerne également les utilisations des adsorbants zéolithiques décrits ci-dessus comme agents d'adsorption susceptibles de remplacer avantageusement les agents d'adsorption décrits dans la littérature, à base de cristaux conventionnels de zéolithe de type FAU, comprenant du baryum ou du baryum et du potassium, ou à base de cristaux conventionnels de zéolithe de type FAU comprenant du baryum ou du baryum et du potassium, et notamment dans les utilisations listées ci-dessous :

- séparation de coupes d'isomères aromatiques en C8 et notamment des xylènes,
- séparation d'isomères de toluène substitué tels que nitrotoluène, diéthyltoluène, toluènediamine, et autres,
- séparation des crésols,
- séparation des alcools polyhydriques, tels que les sucres.

L'adsorbant zéolithique selon la présente invention présente notamment à la fois une résistance mécanique tout particulièrement adaptée et une capacité d'adsorption également tout particulièrement adaptée pour être utilisé dans les procédés de séparation des isomères de xylènes en phase gaz ou en phase liquide.

Ainsi, et selon un autre objet, la présente invention concerne un procédé de séparation des isomères de xylènes en phase gaz ou en phase liquide mettant en oeuvre au moins un adsorbant zéolithique tel que défini précédemment, et de préférence dans lequel les cristaux de zéolithe(s) de l'adsorbant zéolithique sont préparés par synthèse directe mettant en oeuvre un ou plusieurs agents structurants ou gabarits sacrificiels.

L'invention concerne notamment un procédé de séparation de para-xylène à partir de coupes d'isomères aromatiques à 8 atomes de carbone, utilisant, comme agent d'adsorption du para-xylène, un adsorbant zéolithique tel que défini précédemment, et notamment un adsorbant zéolithique comprenant du baryum et/ou du potassium présentant une importante surface externe caractérisée par adsorption d'azote, typiquement supérieure à 20 $m^2.g^{-1}$, de préférence supérieure à 30 $m^2.g^{-1}$, et de préférence encore comprise entre 30 $m^2.g^{-1}$ et 200 $m^2.g^{-1}$, et plus préférentiellement comprise entre 30 $m^2.g^{-1}$ et 150 $m^2.g^{-1}$, mis en oeuvre dans des procédés en phase liquide, mais aussi en phase gazeuse.

On peut ainsi séparer le produit désiré (para-xylène) par chromatographie liquide d'adsorption préparative (en batch), et avantageusement en continu en lit mobile simulé, c'est-à-dire à contre-courant simulé ou à co-courant simulé, et plus particulièrement à contre-courant simulé.

Les conditions opératoires d'une unité industrielle d'adsorption de type contre-courant simulé sont en général les suivantes :

- nombre de lits : 6 à 30,

- nombre de zones : au moins 4 zones de fonctionnement, chacune étant localisées entre un point d'alimentation et un point de soutirage,
- température comprise entre 100°C et 250°C, de préférence entre 150°C et 190°C,
- pression de l'unité industrielle comprise entre la pression de bulle des xylènes à la température du procédé et 3 MPa,
- rapport des débits désorbant/charge compris entre 0,7 et 2,5, par exemple entre 0,9 et 1,8 pour une unité d'adsorption seule (dite "stand alone") et entre 0,7 et 1,4 pour une unité d'adsorption combinée à une unité de cristallisation,
- taux de recyclage compris entre 2 et 12 de préférence entre 2,5 et 6,0.

On pourra sur ce sujet se référer à l'enseignement des brevets US2985589, US5284992 et US5629467.

Les conditions opératoires d'une unité industrielle d'adsorption à co-courant simulé sont en général les mêmes que celles fonctionnant à contre-courant simulé, à l'exception du taux de recyclage qui est en général compris entre 0,8 et 7. On pourra sur cet aspect se référer aux brevets US 4 402 832 et US 4 498 991.

Le solvant de désorption peut être tout désorbant connu de l'homme du métier et dont le point d'ébullition est inférieur à celui de la charge, tel que le toluène mais aussi un désorbant dont le point d'ébullition est supérieur à celui de la charge, tel que le *para*-diéthylbenzène (PDEB). La sélectivité des adsorbants selon l'invention pour l'adsorption du *para*-xylène contenu dans des coupes aromatiques en C8 est optimale lorsque leur perte au feu mesurée à 950°C est de préférence inférieure ou égale à 7,7%, de préférence comprise entre 0 et 7,7%, de manière très préférée entre 3,0 et 7,7%, de manière plus préférée entre 3,5 et 6,5 % et de manière encore plus préférée entre 4,5 et 6%.

Le procédé de séparation de *para*-xylène selon la présente invention présente des avantages essentiels, par rapport à ceux connus de l'art antérieur, et parmi ceux-ci, notamment celui d'offrir un compromis tout particulièrement intéressant entre capacité d'adsorption satisfaisante grâce en particulier à un taux de phase non zéolithique réduit typiquement de 6% à 12 % en poids de liant, par rapport au poids total de l'adsorbant et une bonne résistance mécanique, mesurée par la méthode Shell série SMS1471-74 adaptée pour des agglomérés de taille inférieure à 1,6 mm, typiquement comprise entre 1,5 MPa et 4 MPa, de préférence entre 1,7 MPa et 4 MPa, de préférence encore entre 1,8 MPa et 4 MPa et de manière tout à fait préférée entre 2 MPa et 4 MPa.

En outre, il a été remarqué que la surface externe (typiquement supérieure à 20 $m^2.g^{-1}$ comme indiqué précédemment) permet de réduire le temps de transport vers les micropores, conduisant à un transfert de matière significativement amélioré par rapport à l'art antérieur.

Par ailleurs, une étape de zéolithisation, souvent recommandée dans l'art antérieur pour maximiser la teneur en zéolithe des adsorbants zéolithiques, n'étant pas nécessaire avec les adsorbants zéolithiques de la présente invention, le procédé de préparation desdits adsorbants zéolithiques présente de nombreux avantages très recherchés, parmi lesquels on peut citer les gains de coûts, les gains de temps de procédés, une réduction significative de la dégradation des cristaux par les solutions basiques, comme cela est souvent le cas lors des opérations de zéolithisation notamment lors de l'utilisation de cristaux de taille nanométrique.

Un autre avantage est de pouvoir disposer de cristaux de taille micrométrique (typiquement comprise entre 1 $\mu$m et 20 $\mu$m, de préférence encore comprise entre 1,5 $\mu$m et 20 $\mu$m, plus préférentiellement entre 1,8 $\mu$m et 10 $\mu$m, mieux encore entre 2 $\mu$m et 10 $\mu$m, et de préférence encore entre 2 $\mu$m et 8 $\mu$m) qui sont plus facilement manipulables, rendant ainsi la fabrication d'adsorbants plus aisée.

Ainsi, les adsorbants zéolithiques de l'invention présentent notamment des propriétés de transfert de matière améliorées tout en maintenant des propriétés optimales de sélectivité vis-à-vis du *para*-xylène et de capacité d'adsorption maximales, et en conservant une résistance mécanique élevée pour une utilisation dans un procédé de séparation du *para-xylène* en phase liquide, de préférence de type contre-courant simulé.

TECHNIQUES DE CARACTÉRISATION

**Granulométrie des cristaux zéolithiques - Détection des mésopores**

L'estimation du diamètre moyen en nombre des cristaux de zéolithe FAU contenus dans les adsorbants zéolithiques selon l'invention est réalisée par observation au microscope électronique à balayage (MEB).

Afin d'estimer la taille des cristaux de zéolithe sur les échantillons, on effectue un ensemble de clichés à un grossissement d'au moins 5000. On mesure ensuite le diamètre d'au moins 200 cristaux à l'aide d'un logiciel dédié. La précision est de l'ordre de 3%.

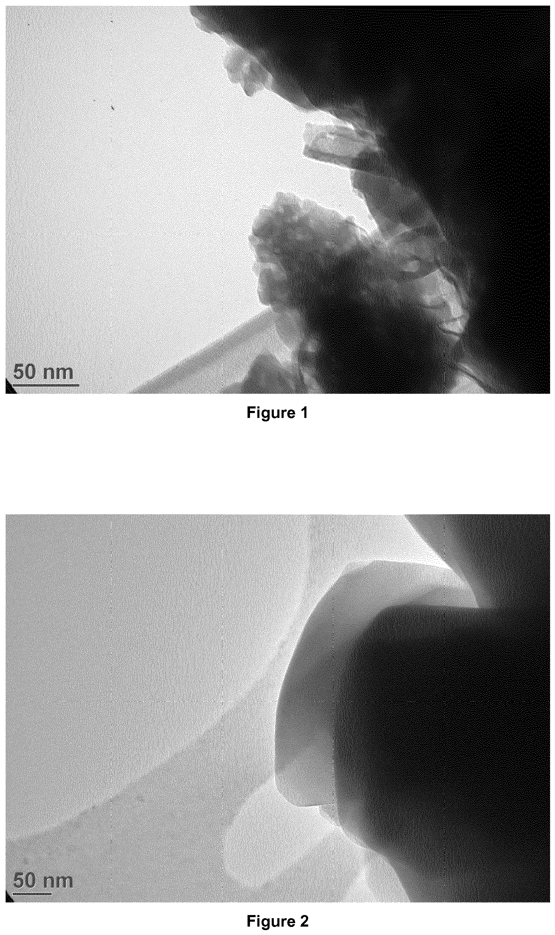

Comme indiqué dans US 7785563, le MET permet en outre de vérifier si les cristaux zéolithiques de l'adsorbant de la présente invention sont des cristaux de zéolithe pleins (i.e. non mésoporeux) ou des agrégats de cristaux de zéolithes pleins ou des cristaux mésoporeux (cf. la comparaison des clichés MET de la Figure 1, où la mésoporosité est clairement visible et la Figure 2 qui montrent des cristaux pleins). L'observation MET permet ainsi de visualiser la présence ou l'absence des mésopores. De manière préférée, les adsorbants du procédé selon l'invention contiennent très majoritairement, i.e. typiquement plus de 80% et de préférence plus de 90% en nombre des cristaux zéolithiques mésoporeux et non des cristaux pleins. Cette analyse statistique est effectuée avantageusement par analyse d'au moins 50 clichés MET ou MEB (MEB sur sections d'échantillons obtenues par polissage ionique).

**Analyse chimique des adsorbants zéolithiques - rapport Si/Al et taux d'échange** :

Une analyse chimique élémentaire du produit final obtenu à l'issue des étapes a) à e) décrites précédemment, peut être réalisée selon différentes techniques analytiques connues de l'homme du métier. Parmi ces techniques, on peut citer la technique d'analyse chimique par fluorescence de rayons X telle que décrite dans la norme NF EN ISO 12677 : 2011 sur un spectromètre dispersif en longueur d'onde (WDXRF), par exemple Tiger S8 de la société Bruker.

La fluorescence X est une technique spectrale non destructive exploitant la photoluminescence des atomes dans le domaine des rayons X, pour établir la composition élémentaire d'un échantillon. L'excitation des atomes généralement par un faisceau de rayons X ou par bombardement avec des électrons, génère des radiations spécifiques après retour à l'état fondamental de l'atome. Le spectre de fluorescence X a l'avantage de dépendre très peu de la combinaison chimique de l'élément, ce qui offre une détermination précise, à la fois quantitative et qualitative. On obtient de manière classique après étalonnage pour chaque oxyde une incertitude de mesure inférieure à 0,4% en poids.

Ces analyses chimiques élémentaires permettent à la fois de vérifier le rapport atomique Si/Al de la zéolithe utilisée lors de la préparation de l'adsorbant, ainsi que le rapport atomique Si/Al de l'adsorbant et de vérifier la qualité de l'échange ionique décrit à l'étape b) et à l'étape optionnelle c). Dans la description de la présente invention, l'incertitude de mesure du rapport atomique Si/Al est de $\pm$ 5%.

La qualité de l'échange ionique est liée au nombre de mole d'oxyde de sodium, $Na_2O$, restant dans l'adsorbant zéolithique après échange. Plus précisément, le taux d'échange par les ions baryum est estimé en évaluant le rapport entre le nombre de moles d'oxyde de baryum, BaO, et le nombre de moles de l'ensemble (BaO + $Na_2O$). De même, le taux d'échange par les ions baryum et/ou potassium est estimé en évaluant le rapport entre le nombre de moles de l'ensemble oxyde de baryum + oxyde de potassium (BaO + $K_2O$) et le nombre de moles de l'ensemble (BaO + $K_2O$ + $Na_2O$). Il est à noter que les teneurs en différents oxydes sont donnés, en pourcentage en poids par rapport au poids total de l'adsorbant zéolithique anhydre.

**Granulométrie des adsorbants zéolithiques** :

La détermination du diamètre moyen en volume des adsorbants zéolithiques obtenus à l'issus de l'étape a) d'agglomération et de mise en forme est effectuée par analyse de la distribution granulométrique d'un échantillon d'adsorbant par imagerie selon la norme ISO 13322-2:2006, en utilisant un tapis roulant permettant à l'échantillon de passer devant l'objectif de la caméra.

Le diamètre moyen en volume est ensuite calculé à partir de la distribution granulométrique en appliquant la norme ISO 9276-2:2001. Dans le présent document, on emploie l'appellation « diamètre moyen en volume» ou bien « taille » pour les adsorbants zéolithiques. La précision est de l'ordre de 0,01 mm pour la gamme de taille d'adsorbants de l'invention.

**Résistance mécanique des adsorbants zéolithiques** :

La résistance à l'écrasement d'un lit d'adsorbants zéolithiques tels que décrits dans la présente invention est caractérisée selon la méthode Shell série SMS1471-74 (Shell Method Series SMS1471-74 « Détermination of Bulk Crushing Strength of Catalysts. Compression-Sieve Method »), associée à l'appareil « BCS Tester » commercialisé par la société Vinci Technologies. Cette méthode, initialement destinée à la caractérisation de catalyseurs de 3 mm à 6 mm est basée sur l'utilisation d'un tamis de 425 $\mu$m qui va permettre notamment de séparer les fines créées lors de l'écrasement. L'utilisation d'un tamis de 425 $\mu$m reste adaptée pour des particules de diamètre supérieur à 1,6 mm, mais doit être adaptée selon la granulométrie des adsorbants que l'on cherche à caractériser.

Les adsorbants de la présente invention, généralement sous forme de billes ou d'extrudés, ont en général un diamètre moyen en volume ou une longueur, i.e. plus grande dimension dans le cas des adsorbants non sphériques, comprise entre 0,2 mm et 2 mm, et en particulier comprise entre 0,2 mm et 0,8 mm et de préférence entre 0,40 mm et 0,65 mm. Par conséquent, un tamis de 100 $\mu$m est utilisé à la place du tamis de 425 $\mu$m mentionné dans la méthode Shell standard SMS1471-74.

Le protocole de mesure est le suivant : un échantillon de 20 $cm^3$ d'adsorbants agglomérés, préalablement tamisé avec le tamis adapté (100 $\mu$m) et préalablement séché à l'étuve pendant au moins 2 heures à 250°C (au lieu de 300°C mentionné dans la méthode Shell standard SMS1471-74), est placé dans un cylindre métallique de section interne connue. Une force croissante est imposée par paliers sur cet échantillon par l'intermédiaire d'un piston, à travers un lit de 5 $cm^3$ de billes d'acier afin de mieux répartir la force exercée par le piston sur les adsorbants agglomérés (utilisation de billes de 2 mm de diamètre pour des particules de forme sphérique de diamètre strictement inférieur à 1,6 mm). Les fines obtenues aux différents paliers de pression sont séparées par tamisage (tamis adapté de 100 μm) et pesées.

La résistance à l'écrasement en lit est déterminée par la pression en mégaPascal (MPa) pour laquelle la quantité de fines cumulées passant à travers le tamis s'élève à 0,5% pondéral de l'échantillon. Cette valeur est obtenue en traçant sur un graphique la masse de fines obtenue en fonction de la force appliquée sur le lit d'adsorbant et en interpolant à 0,5 % massique de fines cumulées. La résistance mécanique à l'écrasement en lit est typiquement comprise entre quelques centaines de kPa et quelques dizaines de MPa et généralement comprise entre 0,3 MPa et 3,2 MPa. La précision est de manière classique inférieure à 0,1 MPa.

**Phase non zéolithique des adsorbants zéolithiques** :

Le taux de phase non zéolithique PNZ, par exemple le taux de liant d'agglomération et de toute autre phase amorphe est calculé selon l'équation suivante :

$$PNZ = 100 - \Sigma \, (PZ),$$

où PZ représente la somme des quantités des fractions zéolithiques X au sens de l'invention.

**Quantité massique des fractions zéolithiques des adsorbants zéolithiques**

La quantité massique des fractions zéolithiques est mesurée par analyse par diffraction de rayons X, connue de l'homme du métier sous l'acronyme DRX. Cette analyse est réalisée sur un appareil de la marque Bruker, puis la quantité des fractions zéolithiques est évaluée à partir des intensités de pic des diffractogrammes en prenant comme référence les intensités de pic d'une référence appropriée (zéolithe de même nature chimique supposée 100% cristalline dans des conditions de traitements cationiques identiques à celles l'adsorbant considéré). Les pics, permettant de remonter à la cristallinité, sont les pics les plus intenses de la zone angulaire $2\theta$ comprise entre 9° et 37°, à savoir les pics observés dans les plages angulaires $2\theta$ comprises respectivement entre 11° et 13°, entre 22° et 26° et entre 31° et 33°.

**Volume microporeux, surface externe et diamètre des mésopores**

La cristallinité des adsorbants zéolithiques de l'invention est également évaluée par mesure de leur volume microporeux en le comparant à celui d'une référence appropriée (zéolithe 100% cristalline dans des conditions de traitements cationiques identiques ou zéolithe théorique). Ce volume microporeux est déterminé à partir de la mesure de l'isotherme d'adsorption de gaz, tel que l'azote, à sa température de liquéfaction.

Préalablement à l'adsorption, l'adsorbant zéolithique est dégazé entre 300°C et 450°C pendant une durée comprise entre 9 heures et 16 heures, sous vide (P < $6,7.10^{-4}$ Pa). La mesure de l'isotherme d'adsorption d'azote à 77K est ensuite effectuée sur un appareil de type ASAP 2020 M de Micromeritics, en prenant au moins 35 points de mesure à des pressions relatives de rapport $P/P_0$ compris entre 0,002 et 1.

Le volume microporeux ainsi que la surface externe sont déterminés à partir de l'isotherme obtenue, par la méthode du t-plot en appliquant la norme ISO 15901-3:2007 et en calculant l'épaisseur statistique t par l'équation de Harkins-Jura. Le volume microporeux et la surface externe sont obtenus par régression linéaire sur les points du t-plot compris entre 0,45 nm et 0,57 nm, respectivement à partir de l'ordonnée à l'origine et de la pente de la régression linéaire. Le volume microporeux évalué s'exprime en $cm^3$ d'adsorbat liquide par gramme d'adsorbant anhydre. La surface externe s'exprime en $m^2$ par gramme d'adsorbant anhydre.

L'interprétation de l'isotherme d'adsorption d'azote à 77K par la méthode de Barrett-Joyner-Halenda (méthode BJH, proposée en 1951) permet en outre d'obtenir la distribution de taille de pores, et notamment la distribution des mésopores. La distribution en volume de taille de mésopores est représentée par la courbe $d$V/$d$Dm en fonction du diamètre moyen des pores *Dm*.

La largeur à mi-hauteur de la distribution en volume $d$V/$d$Dm est donnée par la différence entre les deux diamètres moyens pour lesquels la valeur $d$V/$d$Dm serait égale à la moitié de sa valeur maximale $f_{max}$, au sommet du pic. Ces deux diamètres moyens sont obtenus par interpolation entre les points recherchés de part et d'autre du mode, pour lesquels $d$V/$d$Dm encadre la valeur $f_{max}$/2. Il s'agit de la largeur à mi-hauteur ou FWHM (acronyme anglais pour « Full width at half maximum ») d'une distribution f(x) de valeur maximale est $f_{max}$.

**Volume macroporeux et mésoporeux et densité de grain**

Les volumes macroporeux et mésoporeux ainsi que la densité de grain sont mesurés par porosimétrie par intrusion de mercure. Un porosimètre à mercure type Autopore® 9500 de Micromeritics est utilisé pour analyser la répartition du volume poreux contenu dans les macropores et dans les mésopores.

La méthode expérimentale, décrite dans le manuel opératoire de l'appareil faisant référence à la norme ASTM D4284-83, consiste à placer un échantillon d'adsorbant (matériau granulaire zéolithique à mesurer) (de perte au feu connue) préalablement pesé, dans une cellule du porosimètre, puis, après un dégazage préalable (pression d'évacuation de 30 $\mu$m Hg pendant au moins 10 min), à remplir la cellule avec du mercure à une pression donnée (0,0036 MPa), et ensuite à appliquer une pression croissante par palier jusqu'à 400 MPa afin de faire pénétrer progressivement le mercure dans le réseau poreux de l'échantillon.

La relation entre la pression appliquée et le diamètre apparent des pores est établie en supposant des pores cylindriques, un angle de contact entre le mercure et la paroi des pores de 140° et une tension de surface du mercure de 485 dynes/cm. La quantité cumulée de mercure introduite en fonction de la pression appliquée est enregistrée. On fixe à 0,2 MPa la valeur à partir de laquelle le mercure remplit tous les vides inter-granulaires, et on considère qu'au delà le mercure pénètre dans les pores du matériau granulaire. Le volume de grain (Vg) est alors calculé en soustrayant le volume cumulé de mercure à cette pression (0,2 MPa) au volume de la cellule du porosimètre, et en divisant cette différence par la masse du matériau granulaire équivalent anhydre, c'est-à-dire la masse dudit matériau corrigée de la perte au feu.

La densité de grain est l'inverse du volume de grain (Vg), et s'exprime en gramme d'adsorbant anhydre par $cm^3$.

Le volume macroporeux du matériau granulaire est défini comme étant le volume cumulé de mercure introduit à une pression comprise entre 0,2 MPa et 30 MPa, correspondant au volume contenu dans les pores de diamètre apparent supérieur à 50 nm. Le volume mésoporeux du matériau granulaire est défini comme étant le volume cumulé de mercure introduit à une pression comprise entre 30 MPa et 400 MPa.

Dans le présent document, les volumes macroporeux et mésoporeux des adsorbants zéolithiques, exprimés en $cm^3.g^{-1}$, sont ainsi mesurés par intrusion de mercure et rapportés à la masse de l'échantillon en équivalent anhydre, c'est-à-dire la masse dudit matériau corrigée de la perte au feu.

### Perte au feu des adsorbants zéolithiques :

La perte au feu est déterminée en atmosphère oxydante, par calcination de l'échantillon à l'air à une température de 950°C $\pm$ 25°C, comme décrit dans la norme NF EN 196-2 (avril 2006). L'écart type de mesure est inferieur à 0,1%.

### Exemple A : Synthèse de zéolithe FAU à porosité hiérarchisée

La zéolithe FAU à haute surface externe est synthétisée de manière directe d'après l'article Inayat et coll. (Angew. Chem. Int. Ed., (2012), 51, 1962-1965).

### *Étape 1) : Préparation du gel de croissance dans réacteur agité avec vis d'Archimède à 300 tr.min$^{-1}$.*

Dans un réacteur en inox muni d'une double enveloppe chauffante, d'une sonde température et d'un agitateur, on prépare un gel de croissance en mélangeant une solution d'aluminate contenant 119 g d'hydroxyde de sodium (NaOH) à, 128 g d'alumine trihydratée ($Al_2O_3$, $3H_2O$, contenant 65,2% en poids d'$Al_2O_3$) et 195,5 g eau à 25°C en 25 minutes avec une vitesse d'agitation de 300 tr.min$^{-1}$ dans une solution de silicate contenant 565,3 g de silicate de sodium, 55,3 g de NaOH et 1997,5 g d'eau à 25°C.

La stoechiométrie du gel de croissance est la suivante : 3,48 $Na_2O$ / $Al_2O_3$ / 3,07 $SiO_2$ / 180 $H_2O$. L'homogénéisation du gel de croissance est réalisée sous agitation à 300 tr.min$^{-1}$, pendant 25 minutes à 25°C.

### *Étape 2) : Introduction dans le milieu réactionnel de l'agent structurant*

On introduit dans le milieu réactionnel 27,3 g de solution de TPOAC à 60% dans le MeOH avec une vitesse d'agitation de 300 tr.min$^{-1}$ (ratio molaire TPOAC/$Al_2O_3$ = 0,04). Après 5 minutes d'homogénéisation, on baisse la vitesse d'agitation à 50 tr.min$^{-1}$.

### *Étape 3) : Phase de mûrissement*

On maintient le milieu réactionnel agité à 50 tr.min$^{-1}$ à 25°C, pendant 22 heures, puis on démarre la cristallisation.

### *Étape 4) : Cristallisation*

On maintient la vitesse d'agitation à 50 tr.min$^{-1}$, et on fixe la consigne de la double enveloppe du réacteur à 80°C afin que le milieu réactionnel monte en température à 75°C en 80 minutes. Après 72 heures de palier à 75°C, on refroidit le milieu réactionnel en faisant circuler de l'eau froide dans la double enveloppe pour stopper la cristallisation.

### *Étape 5) : Filtration / lavage*

Les solides sont récupérés sur fritté puis lavés avec de l'eau permutée jusqu'à pH neutre.

### *Étape 6)* : Séchage / Calcination

Afin de caractériser le produit, le séchage est réalisé en étuve à 90°C pendant 8 heures, la perte au feu du produit séché est de 22 % en poids.

La calcination du produit séché nécessaire pour libérer à la fois la microporosité (eau) et la mésoporosité en éliminant l'agent structurant est effectuée avec le profil de température suivant : 30 minutes de montée à 200°C, puis 1 heure de palier à 200°C, puis 3 heures de montée à 550°C, et enfin 1,5 heures de palier à 550°C.

Le volume microporeux et la surface externe mesurés selon la méthode du t-plot à partir de l'isotherme d'adsorption d'azote à 77K après dégazage sous vide à 400°C pendant 10 heures sont respectivement 0,260 $cm^3.g^{-1}$ et 90 $m^2.g^{-1}$. Le diamètre moyen en nombre des cristaux de la zéolithe mésoporeuse (ou à porosité hiérarchisée) ainsi obtenue est de 4,5 $\mu$m et le rapport Si/Al est égal à 1,24.

Dans ce qui suit une masse exprimée en équivalent anhydre signifie une masse de produit diminuée de sa perte au feu.

### Exemple 1 : (comparatif)

***Préparation d'un adsorbant zéolithique sous forme de billes avec une zéolithe de type XPH, taille des cristaux de zéolithe de 4,5 $\mu$m et un liant de type kaolin tel que le taux de phase non zéolithique (PNZ) de l'adsorbant final est égal à 16% en poids par rapport au poids total de l'adsorbant.***

On prépare un mélange homogène constitué de 1600 g équivalent anhydre de cristaux de zéolithe X synthétisée selon le mode opératoire de l'exemple A (taille des cristaux 4,5 $\mu$m), de 350 g équivalent anhydre de kaolin, de 130 g de silice colloïdale vendue sous la dénomination commerciale de Klebosol® 30 (contenant 30% en poids de $SiO_2$ et 0,5% de $Na_2O$) ainsi que de la quantité d'eau qui permet l'agglomération du mélange selon des techniques de mise en forme de billes, comme par exemple assiette granulatrice.

On forme des billes de distribution comprise entre 0,3 mm et 0,8 mm et dont le diamètre moyen en volume est de 0,55 mm de diamètre. Les billes sont séchées une nuit en étuve ventilée à 80°C. Elles sont ensuite calcinées pendant 2 heures à 550°C sous balayage à l'azote, puis 2 heures à 550°C sous balayage à l'air sec décarbonaté.

L'échange au baryum est ensuite opéré avec une concentration de solution de chlorure de baryum, $BaCl_2$, 0,7M à 95°C en 4 étapes. A chaque étape, le ratio volume de solution sur masse de solide est de 20 ml/g et l'échange est poursuivi pendant 4 heures à chaque fois. Entre chaque échange, le solide est lavé plusieurs fois de manière à le débarrasser des excédents de sel. Il est ensuite séché à 80 °C pendant 2 h puis activé à 250 °C pendant 2 h sous courant d'azote.

Le taux d'échange en baryum mesuré par WDXRF, comme décrit plus haut dans les techniques analytiques, est de 97 % et la perte au feu (mesurée à 900°C) est de 5,5%. Le volume microporeux et la surface externe mesurés selon la méthode du t-plot à partir de l'isotherme d'adsorption d'azote à 77K après dégazage sous vide à 400°C pendant 10 heures sont respectivement 0,192 $cm^3.g^{-1}$ et 70 $m^2.g^{-1}$.

Le volume total contenu dans les macropores et les mésopores (somme du volume macroporeux et du volume mésoporeux) mesuré par intrusion de mercure, est de 0,31 $cm^3.g^{-1}$. Le ratio (volume macroporeux)/(volume macroporeux + volume mésoporeux) est égal à 0,65.

Le taux de phase non zéolithique de l'adsorbant échangée à 97% en baryum est de 16% en poids par rapport au poids total de l'adsorbant.

### Exemple 2 :

***Adsorbant avec cristaux de XPH de taille 4,5 $\mu$m et un liant d'agglomération de type kaolin tel que le taux de phase non zéolithique de l'adsorbant final est compris entre 4% et 20% en poids par rapport au poids total de l'adsorbant.***

L'exemple 1 est reproduit en faisant varier la teneur en liant d'agglomération afin d'obtenir des adsorbants dont le taux de phase non zéolithique après échange varie entre 4% et 20%. Les adsorbants sont soumis aux mêmes traitements que dans l'exemple 1. Les résultats sont rassemblés dans le Tableau 1 ci-dessous :

**-- Tableau 1 --**

| Ex. | Taux de PNZ (%) | REL (Mpa) | $V_{micro}$ par t-plot ($cm^3.g^{-1}$) |
|---|---|---|---|
| comparatif | 4 | 0,5 | 0,220 |
| comparatif | 5 | 1,0 | 0,211 |
| selon l'invention | 6 | 1,6 | 0,215 |
| selon l'invention | 8 | 1,8 | 0,211 |
| selon l'invention | 10 | 2,0 | 0,206 |
| selon l'invention | 12 | 2,2 | 0,202 |
| comparatif | 16 | 2,4 | 0,192 |
| comparatif | 20 | 3,0 | 0,183 |

Un test de perçage (chromatographie frontale) est ensuite réalisé sur une sélection de 6 adsorbants pour évaluer leur efficacité. Les adsorbants à 4% et 5% de PNZ ne sont pas testés, car de tels adsorbants ne pourraient pas être utilisés dans l'application de séparation du paraxylène en raison de leur faible résistance mécanique. La quantité d'adsorbant utilisée pour ce test est d'environ 34 g. La perte au feu (PAF) est fixée entre 5,4% et 5,6%.

Le mode opératoire pour obtenir les courbes de perçage est le suivant :

- remplissage de la colonne par le tamis et mise en place dans le banc de test ;
- remplissage par le solvant à température ambiante ;
- montée progressive à 175°C sous flux de solvant (5 $cm^3.min^{-1}$) ;
- injection de solvant à 30 $cm^3.min^{-1}$ lorsque la température d'adsorption (175°C) est atteinte ;
- permutation solvant/charge pour injecter la charge (30 $cm^3.min^{-1}$) ;
- collecte et analyse de l'effluent du perçage ; l'injection de la charge sera maintenue jusqu'à ce que la concentration en solvant dans l'effluent soit nulle.

Le solvant utilisé est le para-diéthylbenzène. La composition de la charge est la suivante :

- *Para*-xylène : 45% poids,
- *Méta*-xylène : 45% poids,
- Iso-octane : 10% poids (celui-ci est utilisé comme traceur pour l'estimation des volumes non-sélectifs et n'intervient pas dans la séparation)

La pression est suffisante pour que la charge reste en phase liquide à la température d'adsorption, soit 1 MPa. La vitesse superficielle est de 1,3 cm/s.

La sélectivité du *para*-xylène par rapport au *méta-xylène* est calculée à partir des quantités adsorbées de chaque composé, ces dernières étant déterminées par bilan matière à partir des premiers moments des courbes de perçage de l'ensemble des constituants présents dans l'effluent. L'évaluation de la qualité du transfert de matière se fait par l'estimation des HEPT à partir des courbes de perçage du *para-xylène.* Les résultats sont consignés dans le Tableau 2 ci-dessous :

**-- Tableau 2 --**

| Exemple | Taux de PNZ (%) | Capacité d'adsorption de xylènes ($cm^3.g^{-1}$) | Transfert du PX (= HEPT) |
|---|---|---|---|
| selon l'invention | 6 | 0,205 | 4,4 |
| selon l'invention | 8 | 0,200 | 4,6 |
| selon l'invention | 10 | 0,194 | 4,7 |
| selon l'invention | 12 | 0,189 | 5,1 |
| comparatif | 16 | 0,181 | 5,4 |
| comparatif | 20 | 0,172 | 6,8 |

Dans le tableau ci-dessus :

- la capacité d'adsorption de xylènes est exprimée en $cm^3$ de C8-aromatiques adsorbé par gramme d'adsorbant ;
- « PX » signifie *para*-xylène ; et enfin
- « HEPT » représente la hauteur équivalente de plateaux théoriques et est exprimée en cm.

Les adsorbants comprenant 16% et 20% en poids de PNZ présentent une perte de capacité d'adsorption supérieure à 10% par rapport à l'adsorbant de capacité d'adsorption de xylènes la plus élevée (0,205 $cm^3.g^{-1}$). Par ailleurs l'augmentation de la résistance diffusionnelle au transfert du PX (HEPT) est de plus en plus prononcée au-delà de 12% de PNZ.

**Revendications**

1. Adsorbant zéolithique comprenant au moins une zéolithe FAU à porosité hiérarchisée, c'est-à-dire possédant à la fois des micropores et des mésopores et comprenant du baryum ou du baryum et du potassium, adsorbant zéolithique pour lequel :

   - la surface externe, mesurée par adsorption d'azote et calculée par la méthode du t-plot à partir de l'isotherme d'adsorption d'azote à une température de 77K, après dégazage sous vide, c'est-à-dire une pression P<6,7 $10^{-4}$Pa, à une température comprise entre 300°c et 450°C pendant une durée allant de 9 heures à 16 heures, est supérieure à 20 $m^2.g^{-1}$, de préférence supérieure à 30 $m^2.g^{-1}$, et de préférence encore comprise entre 30 $m^2.g^{-1}$ et 200 $m^2.g^{-1}$, et plus préférentiellement comprise entre 30 $m^2.g^{-1}$ et 150 $m^2.g^{-1}$, ladite surface externe étant associée à une population de mésopores de diamètre moyen compris entre 2 nm et 50 nm, et
   - le taux de phase non zéolithique est compris entre 6% et 12% en poids par rapport au poids total de l'adsorbant, de préférence compris entre 6% et 11% en poids par rapport au poids total de l'adsorbant, de préférence encore entre 6% et 10%, en poids par rapport au poids total de l'adsorbant.

2. Adsorbant selon la revendication 1, dans lequel la zéolithe FAU à porosité hiérarchisée de l'adsorbant zéolithique est une zéolithe pour laquelle :

   - le diamètre moyen en nombre des cristaux, mesuré par observation au microscope électronique à balayage est compris entre 1 $\mu$m et 20 $\mu$m, de préférence encore compris entre 1,5 $\mu$m et 20 $\mu$m, plus préférentiellement entre 1,8 $\mu$m et 10 $\mu$m, mieux encore entre 2 $\mu$m et 10 $\mu$m, et de préférence encore entre 2 $\mu$m et 8 $\mu$m, ;
   - la surface externe des cristaux, mesurée par adsorption d'azote, supérieure à 40 $m^2.g^{-1}$, de préférence comprise entre 40 $m^2.g^{-1}$ et 200 $m^2.g^{-1}$, de préférence encore comprise entre 40 $m^2.g^{-1}$ et 150 $m^2.g^{-1}$.

3. Adsorbant zéolithique selon l'une quelconque des revendications 1 ou 2, pour lequel le volume total contenu dans les macropores et les mésopores (somme du volume macroporeux et du volume mésoporeux) mesuré par intrusion de mercure et déterminé selon la norme ASTM D4284-83, est compris entre 0,15 $cm^3.g^{-1}$ et 0,5 $cm^3.g^{-1}$, de préférence compris entre 0,20 $cm^3.g^{-1}$ et 0,40 $cm^3.g^{-1}$ et de manière très préférée compris entre 0,20 $cm^3.g^{-1}$ et 0,35 $cm^3.g^{-1}$.

4. Adsorbant zéolithique selon l'une quelconque des revendications précédentes, présentant un ratio (volume macroporeux)/(volume macroporeux + volume mésoporeux) compris entre 0,2 et 1, de préférence compris entre 0,4 et 0,8.

5. Adsorbant zéolithique selon l'une quelconque des revendications précédentes, dans lequel la fraction massique de zéolithe FAU, la zéolithe FAU étant de préférence une zéolithe de type X, est supérieure ou égale à 88% par rapport au poids total d'adsorbant de la présente invention, le complément à 100% étant constitué de phase non zéolithique.

6. Procédé de préparation d'un adsorbant zéolithique selon l'une quelconque des revendications 1 à 5, ledit procédé comprenant au moins les étapes de :

   a) agglomération de cristaux d'au moins une zéolithe de type FAU à porosité hiérarchisée, c'est-à-dire possédant à la fois des micropores et des mésopores, présentant une surface externe, mesurée par adsorption d'azote, et calculée par la méthode du t-plot à partir de l'isotherme d'adsorption d'azote à une température de 77K, après dégazage sous vide, c'est-à-dire une pression P<6,7 $10^{-4}$Pa, à une température comprise entre 300°c et 450°C pendant une durée allant de 9 heures à 16 heures, supérieure à 40 $m^2.g^{-1}$, de préférence comprise entre 40 $m^2.g^{-1}$ et 200 $m^2.g^{-1}$, de préférence encore comprise entre 40 $m^2.g^{-1}$ et 150 $m^2.g^{-1}$, dont le diamètre moyen en nombre des cristaux mesuré par observation au microscope électronique à balayage est compris entre 1 $\mu$m et 20 $\mu$m de préférence encore compris entre 1,5 $\mu$m et 20 $\mu$m plus préférentiellement 1,8 $\mu$m et

10μm mieux encore entre 2 μm et 10 μm et de préférence encore entre 2 μm et 8 μm avec un liant comprenant de préférence au moins 80% d'argile ou d'un mélange d'argiles et avec jusqu'à 5% d'additifs ainsi qu'avec la quantité d'eau qui permet la mise en forme du matériau aggloméré, puis séchage et calcination des agglomérats ;

b) échange(s) cationique(s) des agglomérats de l'étape a) par mise en contact avec une solution d'ions baryum et/ou d'ions baryum et d'ions potassium ;

c) échange cationique éventuel supplémentaire des agglomérats de l'étape b) par mise en contact avec une solution d'ions potassium ;

d) lavage et séchage des agglomérats obtenus aux étapes b) ou c), à une température comprise entre 50°C et 150°C ; et

e) obtention de l'adsorbant zéolithique selon l'invention par activation des agglomérats obtenus à l'étape d) sous balayage gazeux oxydant et/ou inerte, avec notamment des gaz tels que l'oxygène, l'azote, l'air, un air sec et/ou décarbonaté, un air appauvri en oxygène, éventuellement sec et/ou décarbonaté, à une température comprise entre 100°C et 400°C, de préférence entre 200°C et 300°C pendant 1 à 6 heures.

7. Procédé selon la revendication 6, dans lequel, à l'étape a), la au moins une zéolithe FAU à porosité hiérarchisée est préparée en présence d'un gabarit sacrificiel destiné à être éliminé.

8. Procédé selon la revendication 6 ou la revendication 7, dans lequel, l'agglomération et la mise en forme (étape a) sont réalisées selon une ou plusieurs des techniques choisies parmi extrusion, compactage, agglomération sur assiette granulatrice, tambour granulateur, atomisation et autres.

9. Procédé selon l'une quelconque des revendications 6 à 8, dans lequel les proportions de liant d'agglomération et de zéolithe mises en oeuvre sont de 8 parties à 15 parties en poids de liant pour 92 parties à 85 parties en poids de zéolithe.

10. Procédé selon l'une quelconque des revendications 6 à 9, dans lequel le liant est choisi parmi les argiles et les mélanges d'argiles, les silices, les alumines, les silices colloïdales, les gels d'alumine, et autres, et leurs mélanges.

11. Procédé selon la revendication 10, dans lequel le liant est choisi parmi kaolins, kaolinites, nacrites, dickites, halloysites, attapulgites, sépiolites, montmorillonites, bentonites, illites et métakaolins, ainsi que les mélanges de deux ou plusieurs de ces argiles, en toutes proportions.

12. Procédé selon la revendication 11, dans lequel l'argile ou les argiles sont formulées sous forme de poudres broyées à sec et sélectées, ou sous forme de gel, et dispersées, et éventuellement broyées, de préférence sous forme de gel.

13. Utilisation d'un adsorbant zéolithique selon l'une quelconque des revendications 1 à 5, comme agent d'adsorption dans :

• la séparation de coupes d'isomères aromatiques en C8 et notamment des xylènes,
• la séparation d'isomères de toluène substitué tels que nitrotoluène, diéthyltoluène, toluènediamine, et autres,
• la séparation des crésols,
• la séparation des alcools polyhydriques, tels que les sucres.

14. Procédé de séparation des isomères de xylènes en phase gaz ou en phase liquide mettant en oeuvre au moins un adsorbant zéolithique selon l'une quelconque des revendications 1 à 5.

15. Procédé de séparation selon la revendication 14, qui est un procédé de séparation de *para*-xylène à partir de coupes d'isomères aromatiques à 8 atomes de carbone, utilisant, comme agent d'adsorption du *para*-xylène, un adsorbant zéolithique selon l'une quelconque des revendications 1 à 5.

**Patentansprüche**

1. Zeolith-Adsorptionsmittel, umfassend mindestens einen FAU-Zeolith mit hierarchisierter Porosität, der gleichzeitig Mikroporen und Mesoporen aufweist, und Barium oder Barium und Kalium umfasst, Zeolith-Adsorptionsmittel, wobei:

- die äußere Oberfläche, gemessen durch Stickstoffadsorption, die nach der t-Plot-Methode aus der Stickstoffadsorptionsisotherme bei einer Temperatur von 77 K nach dem Vakuumentgasen, das heißt einem Druck P < 6,7 × 10⁻⁴ Pa bei einer Temperatur zwischen 300 °C und 450 °C für einen Zeitraum von 9 Stunden bis 16 Stunden, berechnet ist, größer als 20 m².g⁻¹, vorzugsweise größer als 30 m².g⁻¹ ist und noch bevorzugter zwischen 30 m².g⁻¹ und 200 m².g⁻¹ und insbesondere zwischen 30 m².g⁻¹ und 150 m².g⁻¹ beträgt, wobei die äußere Oberfläche mit einer Population von Mesoporen mit einem mittleren Durchmesser zwischen 2 nm und 50 nm verbunden ist, und
- der Anteil an nicht zeolithischer Phase zwischen 6 und 12 Gew.%, bezogen auf das Gesamtgewicht des Adsorptionsmittels, vorzugsweise zwischen 6 und 11 Gew.%, bezogen auf das Gesamtgewicht des Adsorptionsmittels, insbesondere zwischen 6 und 10 Gew.%, bezogen auf das Gesamtgewicht des Adsorptionsmittels, beträgt.

2. Zeolith-Adsorptionsmittel nach Anspruch 1, wobei der FAU-Zeolith mit hierarchisierter Porosität des Zeolith-Adsorptionsmittels ein Zeolith ist, wobei:

- der zahlendurchschnittliche Durchmesser der Kristalle, gemessen durch Beobachtung mit einem Rasterelektronenmikroskop, zwischen 1 μm und 20 μm, vorzugsweise zwischen 1,5 μm und 20 μm, noch bevorzugter zwischen 1,8 μm et 10 μm, insbesondere zwischen 2 μm und 10 μm und vorzugsweise zwischen 2 μm und 8 μm beträgt,
- die äußere Oberfläche der Kristalle, gemessen durch Stickstoffadsorption, größer als 40 m².g⁻¹ ist, vorzugsweise zwischen 40 m².g⁻¹ und 200 m².g⁻¹, insbesondere zwischen 40 m².g⁻¹ und 150 m².g⁻¹ beträgt.

3. Zeolith-Adsorptionsmittel nach einem der Ansprüche 1 oder 2, wobei das Gesamtvolumen, das in den Makroporen und den Mesoporen enthalten ist (Summe des makroporösen Volumens und des mesoporösen Volumens), gemessen durch Intrusion von Quecksilber, bestimmt durch die Norm ASTM D4284-83, zwischen 0,15 cm³.g⁻¹ und 0,5 cm³.g⁻¹, vorzugsweise zwischen 0,20 cm³.g⁻¹ und 0,40 cm³.g⁻¹ und insbesondere zwischen 0,20 cm³.g⁻¹ und 0,35 cm³.g⁻¹ beträgt.

4. Zeolith-Adsorptionsmittel nach einem der vorhergehenden Ansprüche, das ein Verhältnis (makroporöses Volumen)/(makroporöses Volumen + mesoporöses Volumen) zwischen 0,2 und 1, vorzugsweise zwischen 0,4 und 0,8, aufweist.

5. Zeolith-Adsorptionsmittel nach einem der vorhergehenden Ansprüche, wobei der Massenanteil an FAU-Zeolith, wobei der FAU-Zeolith vorzugsweise ein Zeolith vom Typ X ist, größer als oder gleich 88 Gew.%, bezogen auf das Gesamtgewicht des Adsorptionsmittels der vorliegenden Erfindung ist, wobei die Ergänzung auf 100 % durch nicht zeolithische Phase gebildet ist.

6. Verfahren zur Herstellung eines Zeolith-Adsorptionsmittels nach einem der Ansprüche 1 bis 5, wobei das Verfahren mindestens die Schritte aufweist des:

a) Agglomerierens von Kristallen von mindestens einem Zeolith des Typs des FAU-Zeoliths mit hierarchisierter Porosität, der gleichzeitig Mikroporen und Mesoporen aufweist, der eine äußere Oberfläche aufweist, die durch Stickstoffadsorption gemessen und nach der t-Plot-Methode aus der Stickstoffadsorptionsisotherme bei einer Temperatur von 77 K nach dem Vakuumentgasen, das heißt einem Druck P < 6,7 × 10⁻⁴ Pa bei einer Temperatur zwischen 300 °C und 450 °C für einen Zeitraum von 9 Stunden bis 16 Stunden, berechnet wird, die größer als 40 m².g⁻¹ ist, vorzugsweise zwischen 40 m².g⁻¹ und 200 m².g⁻¹, insbesondere zwischen 40 m².g⁻¹ und 150 m².g⁻¹ beträgt, wobei der zahlendurchschnittliche Durchmesser der Kristalle, gemessen durch Beobachtung mit einem Rasterelektronenmikroskop, zwischen 1 μm und 20 μm, vorzugsweise zwischen 1,5 μm und 20 μm, noch bevorzugter zwischen 1,8 μm und 10 μm, insbesondere zwischen 2 μm und 10 μm, und vorzugsweise zwischen 2 μm und 8 μm beträgt, mit einem Bindemittel, das vorzugsweise mindestens 80 % Ton oder einer Mischung aus Tonarten umfasst, und mit bis zu 5 % Zusatzstoffen sowie mit der Menge an Wasser, die das Formen des agglomerierten Materials ermöglicht, und dann des Trocknens und Kalzinierens der Agglomerate,
b) Kationenaustauschens der Agglomerate von Schritt a) durch Inkontaktbringen mit einer Lösung von Barium-Ionen und/oder Barium-Ionen und Kalium-Ionen,
c) eventuellen zusätzlichen Kationenaustauschens der Agglomerate von Schritt b) durch Inkontaktbringen mit einer Lösung von Kalium-Ionen;
d) Waschens und Trocknen der Agglomerate, die in den Schritten b) oder c) erhalten werden, bei einer Temperatur zwischen 50 °C und 150 °C und
e) Erhaltens des Zeolith-Adsorptionsmittels nach der Erfindung durch Aktivieren der Agglomerate, die in Schritt d) erhalten werden, unter oxidierender und/oder inerter Gasspülung, insbesondere mit Gasen wie Sauerstoff, Stickstoff, Luft, einer trockenen und/oder entkarbonisierten Luft, einer sauerstoffarmen, eventuell trockenen und/oder entkarbonisierten Luft, bei einer Temperatur zwischen 100 °C und 400 °C, vorzugsweise zwischen 200 °C und 300 °C, für 1 bis 6 Stunden,

wobei die Anteile an Agglomerationsbindemittel und an Zeolith, die eingesetzt werden, 8 bis 15 Gewichtsteile Bindemittel pro 92 bis 85 Gewichtsteile Zeolith betragen.

**7.** Verfahren nach Anspruch 6, wobei in dem Schritt a) der mindestens eine FAU-Zeolith mit hierarchisierter Porosität in Anwesenheit einer Opferschablone hergestellt wird, die dazu bestimmt ist, entfernt zu werden.

**8.** Verfahren nach Anspruch 6 oder 7, wobei das Agglomerieren und das Formen (Schritt a) nach einer oder mehreren der Techniken durchgeführt werden, die aus Extrudieren, Kompaktieren, Agglomerieren auf einer Granulatorplatte, Granulatortrommel, Atomisieren und dergleichen ausgewählt werden.

**9.** Verfahren nach einem der Ansprüche 6 bis 8, wobei das Bindemittel ausgewählt wird aus Tonen und Tongemischen, Siliciumdioxiden, Aluminiumoxiden, kolloidalen Siliciumdioxiden, Aluminiumoxidgelen und dergleichen und Mischungen davon.

**10.** Verfahren nach Anspruch 9, wobei das Bindemittel ausgewählt wird aus Kaolinen, Kaoliniten, Nacriten, Dickiten, Halloysiten, Attapulgiten, Sepioliten, Montmorilloniten, Bentoniten, Illiten und Metakaolinen sowie den Mischungen von zwei oder mehreren dieser Tone in allen Verhältnissen.

**11.** Verfahren nach Anspruch 10, wobei der Ton oder die Tone in Form von trocken gemahlenen Pulvern oder in Gelform, vorzugsweise in Gelform, formuliert und ausgewählt und dispergiert und gegebenenfalls gemahlen werden.

**12.** Verwendung eines Zeolith-Adsorptionsmittel nach einem der Ansprüche 1 bis 5 als Adsorptionsmittel bei:

- der Trennung von Fraktionen von aromatischen C8-Isomeren und insbesondere von Xylolen,
- der Trennung von Isomeren aus substituiertem Toluol, wie Nitrotoluol, Diethyltoluol, Toluoldiamin und dergleichen,
- der Trennung der Kresole,
- der Trennung der polyhydrischen Alkohole, wie die Zucker.

**13.** Verwendung mindestens eines Zeolith-Adsorptionsmittels, wie in einem der Ansprüche 1 bis 5 definiert, für die Trennung von Isomeren von Xylenen in Gasphase oder in Flüssigphase.

**14.** Verwendung nach Anspruch 13 zur Trennung von *para*-Xylol ausgehend von Fraktionen von aromatischen Isomeren mit 8 Kohlenstoffatomen.

**15.** Verfahren zur Trennung von *para*-Xylol, **dadurch gekennzeichnet, dass** es einen Schritt des Adsorbierens in Gasphase oder in Flüssigphase auf einem Zeolith-Adsorptionsmittel, wie in einem der Ansprüche 1 bis 5 definiert, von Fraktionen von aromatischen Isomeren mit 8 Kohlenstoffatomen aufweist.

**Claims**

**1.** Zeolite adsorbent comprising at least one FAU zeolite with hierarchical porosity, possessing both micropores and mesopores, and comprising barium or barium and potassium, for which zeolite adsorbent:

- the outer surface area, measured by nitrogen adsorption, calculated via the t-plot method from the nitrogen adsorption isotherm at a temperature of 77 K, after degassing under vacuum, that is to say a pressure $P < 6.7 \times 10^{-4}$ Pa, at a temperature of between 300°C and 450°C for a time ranging from 9 hours to 16 hours, is greater than 20 $m^2.g^{-1}$, preferably greater than 30 $m^2.g^{-1}$ and more preferably between 30 $m^2.g^{-1}$ and 200 $m^2.g^{-1}$, and more preferentially between 30 $m^2.g^{-1}$ and 150 $m^2.g^{-1}$, said outer surface area being combined with a population of mesopores with a mean diameter of between 2 nm and 50 nm, and
- the content of non-zeolite phase is between 6% and 12% by weight relative to the total weight of the adsorbent, preferably between 6% and 11% by weight relative to the total weight of the adsorbent, more preferably between 6% and 10% by weight relative to the total weight of the adsorbent.

**2.** Adsorbent according to Claim 1, in which the FAU zeolite with hierarchical porosity of the zeolite adsorbent is a zeolite for which:

- the number-average diameter of the crystals, measured by observation with a scanning electron microscope, is between 1 $\mu$m and 20 $\mu$m, more preferably between 1.5 $\mu$m and 20 $\mu$m, more preferentially between 1.8 $\mu$m and 10 $\mu$m, better still between 2 $\mu$m and 10 $\mu$m and more preferably between 2 $\mu$m and 8 $\mu$m,
- the outer surface area of the crystals, measured by nitrogen adsorption, greater than 40 $m^2.g^{-1}$, preferably between 40 $m^2.g^{-1}$ and 200 $m^2.g^{-1}$ and more preferably between 40 $m^2.g^{-1}$ and 150 $m^2.g^{-1}$.

**3.** Zeolite adsorbent according to either of Claims 1 and 2, for which the total volume contained in the macropores and the mesopores (sum of the macropore volume and of the mesopore volume) measured by mercury intrusion, determined according to standard ASTM D4284-83, is between 0.15 $cm^3.g^{-1}$ and 0.5 $cm^3.g^{-1}$, preferably between 0.20 $cm^3.g^{-1}$ and 0.40 $cm^3.g^{-1}$ and very preferably between 0.20 $cm^3.g^{-1}$ and 0.35 $cm^3.g^{-1}$.

**4.** Zeolite adsorbent according to any one of the preceding claims, with a (macropore volume)/(macropore volume + mesopore volume) ratio of between 0.2 and 1, preferably between 0.4 and 0.8.

**5.** Zeolite adsorbent according to any one of the preceding claims, in which the mass fraction of FAU zeolite, the FAU zeolite preferably being a type X zeolite, is greater than or equal to 88% relative to the total weight of adsorbent of the present invention, the remainder to 100% consisting of non-zeolite phase.

**6.** Process for preparing a zeolite adsorbent according to any one of Claims 1 to 5, said process comprising at least the steps of:

a) agglomeration of crystals of at least one FAU-type zeolite with hierarchical porosity, possessing both micropores and mesopores, having an outer surface area measured by nitrogen adsorption and calculated via the t-plot method from the nitrogen adsorption isotherm at a temperature of 77 K, after degassing under vacuum, that is to say a pressure P < 6.7×10$^{-4}$ Pa, at a temperature of between 300°C and 450°C for a time ranging from 9 hours to 16 hours, of greater than 40 $m^2.g^{-1}$, preferably between 40 $m^2.g^{-1}$ and 200 $m^2.g^{-1}$, more preferably between 40 $m^2.g^{-1}$ and 150 $m^2.g^{-1}$, the number-average diameter of the crystals of which, measured by observation with a scanning electron microscope, is between 1 $\mu$m and 20 $\mu$m, more preferably between 1.5 $\mu$m and 20 $\mu$m, more preferentially 1.8 $\mu$m and 10 $\mu$m, better still between 2 $\mu$m and 10 $\mu$m, and more preferably between 2 $\mu$m and 8 $\mu$m, with a binder preferably comprising at least 80% of clay or of a mixture of clays and with up to 5% of additives and also with the amount of water that allows the forming of the agglomerated material, followed by drying and calcination of the agglomerates;
b) cation exchange(s) of the agglomerates from step a) by placing in contact with a solution of barium ions and/or of barium ions and potassium ions;
c) optional additional cation exchange of the agglomerates from step b) by placing in contact with a solution of potassium ions;
d) washing and drying of the agglomerates obtained in steps b) or c), at a temperature of between 50°C and 150°C; and
e) production of the zeolite adsorbent according to the invention by activation of the agglomerates obtained in step d) under a stream of oxidizing and/or inert gas, especially with gases such as oxygen, nitrogen, air, a dry and/or decarbonated air, an oxygen-depleted air, which is optionally dry and/or decarbonated, at a temperature of between 100°C and 400°C, preferably between 200°C and 300°C for 1 to 6 hours;

in which the proportions of agglomeration binder and of zeolite used are from 8 parts to 15 parts by weight of binder per 92 parts to 85 parts by weight of zeolite.

**7.** Process according to Claim 6, in which, in step a), the at least one FAU zeolite with hierarchical porosity is prepared in the presence of a sacrificial template that is intended to be removed.

**8.** Process according to Claim 6 or Claim 7, in which the agglomeration and the forming (step a) are performed according to one or more of the techniques chosen from extrusion, compacting, agglomeration on a granulating plate, granulating drum, atomization, and the like.

**9.** Process according to any one of Claims 6 to 8, in which the binder is chosen from clays and mixtures of clays, silicas, aluminas, colloidal silicas and alumina gels, and the like, and mixtures thereof.

**10.** Process according to Claim 9, in which the binder is chosen from kaolins, kaolinites, nacrites, dickites, halloysites, attapulgites, sepiolites, montmorillonites, bentonites, illites and metakaolins, and also mixtures of two or more of these clays, in all proportions.

**11.** Process according to Claim 10, in which the clay(s) are formulated in the form of dry-ground and selected powders, or in the form of gel, and dispersed, and optionally ground, preferably in the form of gel.

**12.** Use of a zeolite adsorbent according to any one of Claims 1 to 5, as an adsorption agent in:

- the separation of C8 aromatic isomer fractions, and especially xylenes,
- the separation of substituted toluene isomers such as nitrotoluene, diethyltoluene, toluenediamine and the like,
- the separation of cresols,
- the separation of polyhydric alcohols, such as sugars.

**13.** Use of at least one zeolite adsorbent as defined in any one of Claims 1 to 5, for separating xylene isomers in gas phase or in liquid phase.

**14.** Use according to Claim 13, for separating *para*-xylene from aromatic isomer fractions containing 8 carbon atoms.

**15.** Process for separating para-xylene, **characterized in that** it comprises a step of adsorption, in gas phase or in liquid phase, on a zeolite adsorbent as defined in any one of Claims 1 to 5, of aromatic isomer fractions containing 8 carbon atoms.

Figure 1

Figure 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 3558730 A
- US 3558732 A
- US 3626020 A
- US 3663638 A
- US 3960774 A
- US 3878127 A
- US 2985589 A
- FR 2789914
- US 7812208 B
- FR 2925366
- FR 2999098
- US 8283274 B

- CN 1267185 C
- US 20090326308 A
- US 7785563 B
- WO 2013106816 A
- WO 2007043731 A
- EP 170299 A
- US 6743745 B
- US 5284992 A
- US 5629467 A
- US 4402832 A
- US 4498991 A

**Littérature non-brevet citée dans la description**

- Principles of Adsorption and Adsorption Processes. Principes de l'Adsorption et des Procédés d'Adsorption. John Wiley & Sons, 1984, 326, , 407
- **RUTHVEN.** *Principles of Adsorption and Adsorption Processes,* 248-250
- **LI ET COLL. ; JINGXI SHIYOU HUAGONG.** *Primary Analysis on State of Xylene Adsorption Unit,* 2004, vol. 4, 54-55

- **E. P.BARRETT ; L. G. JOYNER ; P. P. HALENDA.** The Determination of Pore Volume and Area Distributions in Porous Substances. I. Computations from Nitrogen Isotherms. *J. Am. Chem. Soc.,* 1951, vol. 73 (1), 373-380
- **INAYAT.** *Angew. Chem. Int. Ed.,* 2012, vol. 51, 1962-1965
- **D. VERBOEKEND.** *Adv. Funct. Mater.,* 2012, vol. 22, 916-928